# EUROPÄISCHE PATENTANMELDUNG

(11) **EP 4 378 920 A1**
(43) Veröffentlichungstag der Anmeldung: **05.06.2024**
(21) Anmeldenummer: 22210976.1
(22) Anmeldetag: 02.12.2022
(51) Int. Cl.: C07C 213/10, C07C 217/06, A61K 31/245, A61K 47/02, A61K 47/06, A61P 23/00

(54) **STABILISIERTE KOHLENSÄUREADDUKTE**

(71) Anmelder: inflamed pharma GmbH, 07745 Jena (DE)
(72) Erfinder: ENGERT, Beatrice, 07745 Jena (DE); TOEPEL, Christina, 07745 Jena (DE)
(74) Vertreter: Schiweck Weinzierl Koch Patentanwälte Partnerschaft mbB

(57) **Zusammenfassung**

Die Erfindung bezieht sich auf die stabilisierten Kohlensäure-Addukte (SKA1), (SKA2), (SKA3) und (SKA4) umfassend Kohlensäure, mindestens ein Amin (AM), optional mindestens ein Salz (S), und Polyvinylpyrrolidin.

## Beschreibung

### TECHNISCHES GEBIET DER ERFINDUNG

Die Erfindung bezieht sich auf die stabilisierten Kohlensäure-Addukte (SKA1), (SKA2), (SKA3) und (SKA4) umfassend Kohlensäure, mindestens ein Amin (AM), optional mindestens ein Salz (S), und Polyvinylpyrrolidin.

### TECHNISCHER HINTERGRUND

WO2006/007835 A2 und WO2019048590 A2 offenbaren Kohlensäure-Addukte von Aminen, die vor allem für pharmazeutisch-medizinische Anwendungen von Interesse sind. Insbesondere für das Kohlensäure-Addukt des Procains ist aus dieser Offenbarung bekannt, dass der Transport des Wirkstoffes, die Bioverfügbarkeit und die Verträglichkeit, unter anderem auch bei Acidosen verbessert wird. Ebenso wird eine im Vergleich zum Procain ausbleibende Hämolyse beobachtet. Ursache hierfür ist das Vorliegen eines Säure-Base Paares aus Amin und Kohlensäure, das eine hohe Pufferkapazität aufweist und gut löslich ist. Es wird außerdem erläutert, dass es von Bedeutung für die Stabilität des Procainiumhydrogencarbonats ist, die Konzentration von basischen Stoffen, wie Procain oder Carbonat etwa in Infusionslösungen desselben, niedrig zu halten. Die Basizität des Carbonates fördert die Verseifung des Procains. Außerdem katalysieren schon geringe Mengen an Carbonat den Zerfall des Procainiumhydrogencarbonats in Procain und Diprocainiumcarbonat. Als eine Möglichkeit zur Stabilisierung des Procainiumhydrogencarbonats oder Lidocainiumhydrogencarbonates wird der Einbau in Mineralsalze, wie Natriumchlorid und/ oder Dextrane, Stärke oder Cellulose zu Clathraten oder Clustern genannt. Weiterhin ist der Zusatz von CO₂ förderlich wodurch der pH-Wert abgesenkt und der Anteil des Procainiumhydrogencarbonats nahezu auf 100 % gebracht wird.

WO2019048590 A2 offenbart zusätzlich, dass durch geeignete Verfahrensschritte u.a. durch Optimierung der CO₂ Konzentration in der Lösung bei der Herstellung, die Lagerstabilität der Kohlensäure-Addukte erhöht werden kann.

Allerdings besteht weiterer Bedarf für weitere Formulierungen für Kohlensäure-Addukte die eine erhöhte Stabilität aufweisen, besser formulierbar sind, das allgemeine Handling erleichtern und/oder auch Mehrentnahmesysteme ermöglichen.

### ZUSAMMENFASSUNG DER ERFINDUNG

Die Erfindung bezieht sich auf ein stabilisiertes Kohlensäure-Addukt (SKA1) umfassend Kohlensäure, mindestens ein Amin (AM) und optional mindestens ein Salz (S),
herstellbar nach einem Verfahren umfassend die Schritte
a) Bereitstellung einer Lösung (A), die mindestens ein Lösungsmittel und in dem mindestens einem Lösungsmittel gelöstes CO₂ umfasst,
optional b) Auflösen einer Base (BA), die nicht dem Amin (AM) entspricht, in der Lösung (A) unter Erhalt der Lösung (A1),
c) Lösen des mindestens einen Amins (AM) in der Lösung (A) oder (A1), unter Erhalt der Lösung (B),
d) Einfrieren der nach Abschluss des Schrittes c) erhaltenen Lösung,
e) Lagerung der in Schritt d) eingefrorenen Lösung bei -100 bis 0 °C für nicht länger als 4 Tage,
g1) Zugabe von Polyvinylpyrrolidon zu dem in den vorangehenden Schritten erhaltenen Kohlensäure-Addukt (KA) unter Erhalt des stabilisierten Kohlensäure-Addukts (SKA1).
wobei der Gehalt an CO₂ in der Lösung, die dem Schritt d) unterzogen wird, mindestens 6 g/l, vorzugsweise mindestens 7 g/l, mehr bevorzugt mindestens 8 g/l, besonders bevorzugt 8,1 g/l bis 9,5 g/l beträgt und das Amin (AM) auch in Form eines Salzes eingesetzt werden kann.

Stabilisiertes Kohlensäure-Addukt (SKA2) umfassend Kohlensäure, mindestens ein Amin (AM) und optional mindestens ein Salz (S),
herstellbar nach einem Verfahren umfassend die Schritte
a) Bereitstellung einer Lösung (A), die mindestens ein Lösungsmittel und in dem mindestens einem Lösungsmittel gelöstes CO₂ umfasst,
optional b) Auflösen einer Base (BA), die nicht dem Amin (AM) entspricht, in der Lösung (A) unter Erhalt der Lösung (A1),
c) Lösen des mindestens einen Amins (AM) in der Lösung (A) oder (A1), unter Erhalt der Lösung (B),
g2) Zugabe von Polyvinylpyrrolidon zu der in Schritt c) erhaltenen Lösung (B) unter Erhalt des stabilisierten Kohlensäure-Addukts (SKA2).
wobei der Gehalt an CO₂ in der Lösung (B), die dem Schritt c) unterzogen wird, mindestens 10 g/l, vorzugsweise mindestens 12 g/l, noch mehr bevorzugt mindestens 14g/l und ganz besonders bevorzugt mindestens 14 to 16 g/l beträgt und das Amin (AM) auch in Form eines Salzes eingesetzt werden kann.

Stabilisiertes Kohlensäure-Addukt (SKA3) umfassend Kohlensäure, mindestens ein Amin (AM) und optional mindestens ein Salz (S),
herstellbar nach einem Verfahren umfassend die Schritte
a) Bereitstellung einer Lösung (A), die mindestens ein Lösungsmittel und in dem mindestens einem Lösungsmittel gelöstes CO₂ umfasst,
optional b) Auflösen einer Base (BA), die nicht dem Amin (AM) entspricht, in der Lösung (A) unter Erhalt der Lösung (A1),
c) Lösen des mindestens einen Amins (AM) in der Lösung (A) oder (A1), unter Erhalt der Lösung (B),
g3) Zugabe von Polyvinylpyrrolidon zu der in Schritt c) erhaltenen Lösung (B) unter Erhalt der Lösung (C),
h) Einfrieren der nach Abschluss des Schrittes g3) erhaltenen Lösung,
j) Lagerung der in Schritt h) eingefrorenen Lösung bei -100 bis 0 °C für nicht länger als 4 Tage,
wobei der Gehalt an CO₂ in der Lösung, die dem Schritt h) unterzogen wird, mindestens 7 g/l, vorzugsweise mindestens 7,5 g/l, beträgt und das Amin (AM) auch in Form eines Salzes eingesetzt werden kann,
wobei die Schritte in der oben aufgeführten Reihenfolge a), optional b), c), g3), h) und j) durchgeführt werden.

Die Erfindung bezieht sich außerdem ein stabilisiertes Kohlensäure-Addukt (SKA4) umfassend Kohlensäure, mindestens ein Amin (AM) und optional mindestens ein Salz (S),
herstellbar nach einem Verfahren umfassend die Schritte
a) Bereitstellung einer Lösung (A), die mindestens ein Lösungsmittel und in dem mindestens einem Lösungsmittel gelöstes CO₂ umfasst,
optional b) Auflösen einer Base (BA), die nicht dem Amin (AM) entspricht, in der Lösung (A) unter Erhalt der Lösung (A1),
c) Lösen des mindestens einen Amins (AM) in der Lösung (A) oder (A1), unter Erhalt der Lösung (B),
d) Einfrieren der nach Abschluss des Schrittes c) erhaltenen Lösung,
e) Lagerung der in Schritt d) eingefrorenen Lösung bei -100 bis 0 °C für nicht länger als 4 Tage,
f) Trocknung der in Schritt e) gelagerten Lösung unter Erhalt von Kohlensäure-Addukt (KA),
k) Trocknung einer mit CO₂ gesättigten Lösung, die das Kohlensäure-Addukt (KA) aus Schritt f) und Polyvinylpyrrolidin umfasst,
wobei der Gehalt an CO₂ in der Lösung, die dem Schritt d) unterzogen wird, mindestens 6 g/l, vorzugsweise mindestens 7 g/l, mehr bevorzugt mindestens 8 g/l, besonders bevorzugt 8,1 g/l bis 9,5 g/l beträgt und das Amin (AM) auch in Form eines Salzes eingesetzt werden kann.

Weiterhin bezieht sich die Erfindung auf das stabilisierte Kohlensäure Addukt (SKA1), (SKA2), (SKA3) und (SKA4) zur Verwendung in einem Verfahren zur Anästhesie, zur Analgesie, zur begleitenden Behandlung von Krebs, zur Entzündungshemmung, zur Behandlung von Amyotropher Lateralsklerose, zur Unterstützung der Wundheilung, insbesondere bei Verbrennungen, offenen Wunden und Narben, zur Behandlung von neurogenen Entzündungen wie Multiple Sklerose, MMN (Multifokale motorische Neuropathie), zur Behandlung von Sinusitis, zur Behandlung von Asthma, zur Behandlung von Rheumatischer Arthritis, zur Behandlung von Alzheimer, zur Behandlung von Demenz, zur Unterstützung der Rekonvaleszenz und zur Unterstützung des Anti-aging, zur Behandlung des Burn-out-Syndroms, zur Behandlung von Arthrose, zur Behandlung von Polyarthritis, zur Behandlung von Schmerzsyndrom und allgemeinen Schmerzen, zur prä- und postoperativen Behandlung (auch bei Knochenbrüchen), zur Präventiv- und Rehabilitationsmedizin, zur Behandlung von Zosterneuralgie, zur begleitenden Behandlung von Zosterneuralgie, zur Behandlung von Erkrankungen der Bauchorgane, wie Leber, Galle, Bauchspeicheldrüse, Darm, zur Behandlung von Magen-Darm-Erkrankungen (Collitis Ulcerosa, Morbus Krohn), zur Behandlung von Morbus Bechterew, zur Behandlung von chron. Schmerzen des Bewegungsapparats, zur Behandlung von Diabetes (Verbesserung der Blutzuckerwerte), zur Reduktion von Ödemen, zur Behandlung von Fatique, Long-COVID, zur Verbesserung der Mikrozirkulation, zur Comedikation zu Opioden.

Das stabilisierte Kohlensäure-Addukt (SKA1),(SKA2), (SKA3) und (SKA4) ermöglicht in einer Ausführungsform ein Mehrentnahmesystem z.B. für neuraltherapeutische Zwecke. Eine solche Anwendung war mit den oben beschriebenen Kohlensäure-Addukten des Stands der Technik bisher nicht möglich. Mehrentnahmesystem bedeutet, dass mehrere Dosen aus einem Behältnis entnehmbar sind. Das stabilisierte Kohlensäure-Addukt (SKA2) für buccale Anwendungen ist bei täglicher Applikation und Aufbewahrung im Kühlschrank etwa 7 Tage stabil. In einer anderen Ausführungsform als Trinklösung ist das stabilisierte Kohlensäure-Addukt (SKA2) 24h bei Raumtemperatur und und bei geschlossener Aufbewahrung im Kühlschrank 10 Monate stabil.

Das stabilisierte Kohlensäure-Addukt (SKA3) und (SKA4) erweist sich als recht stabil und lässt sich in einer Ausführungsform direkt als Feststoffgemisch zu Tabletten verpressen. Alternativ lassen sich aus dem stabilisierte Kohlensäure-Addukt (SKA3) Salben mit wasserhaltigen Grundlagen herstellen. Gegenüber den bekannten Kohlensäure-Addukten aus dem Stand der Technik weist das stabilisierte Kohlensäure-Addukt (SKA3) eine verzögerte Freisetzung auf.

### KURZE BESCHREIBUNG DER FIGUREN

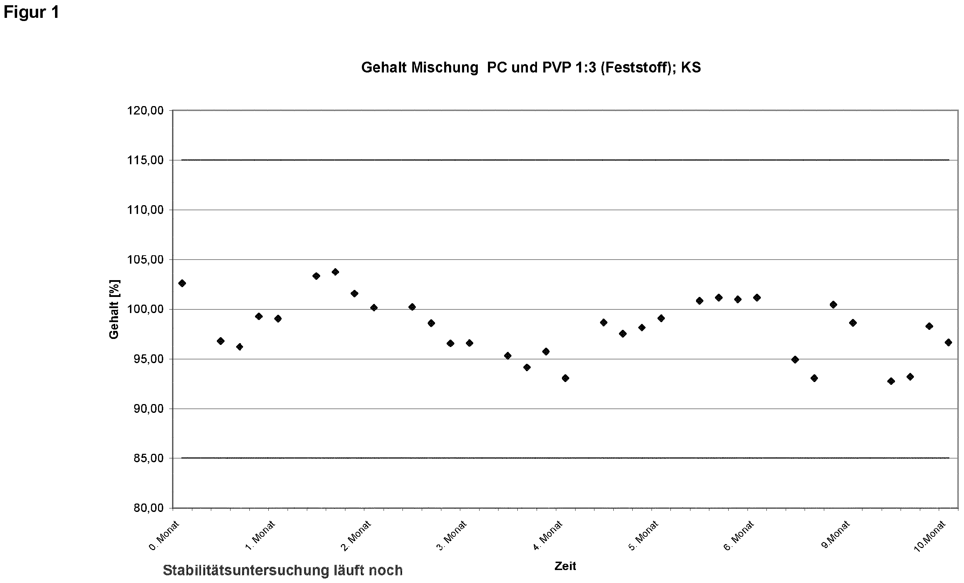

**Fig. 1****:** Stabilität des SKA1 (1:3) bei Lagerung im Kühlschrank

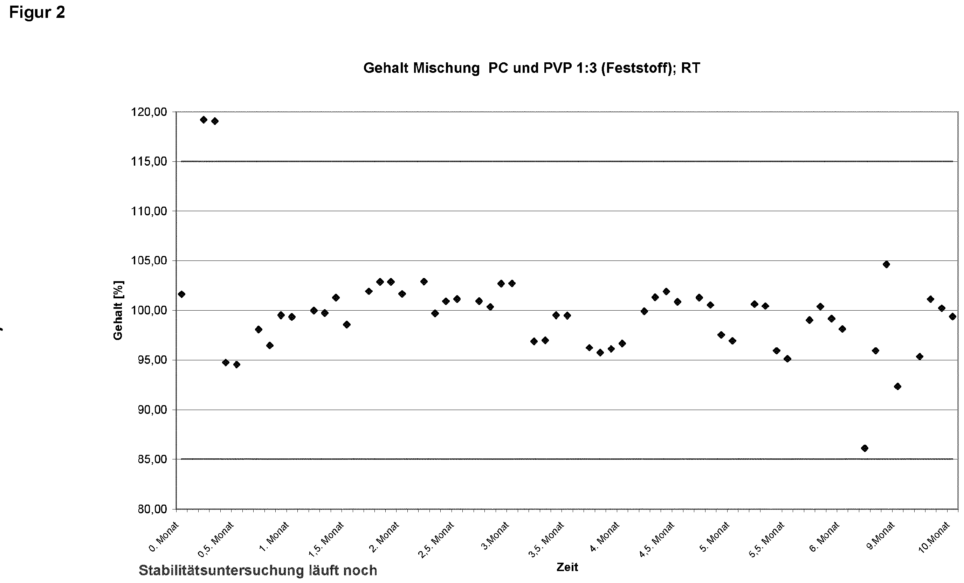

**Fig. 2****:** Stabilität des SKA1 (1:3) bei Lagerung bei Raumtemperatur

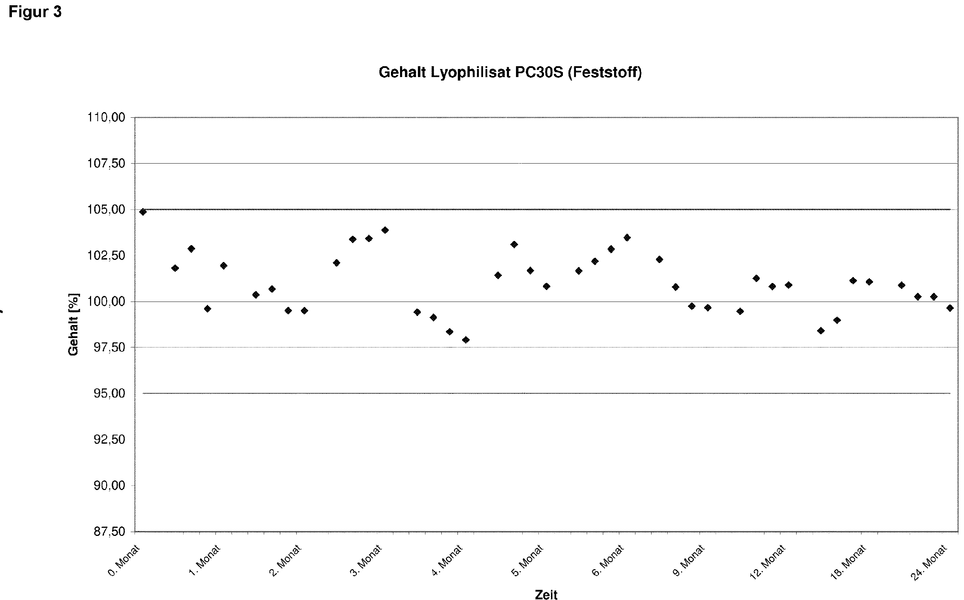

**Fig.** 3: Stabilität SKA3 und SKA4 (mit PVP30)

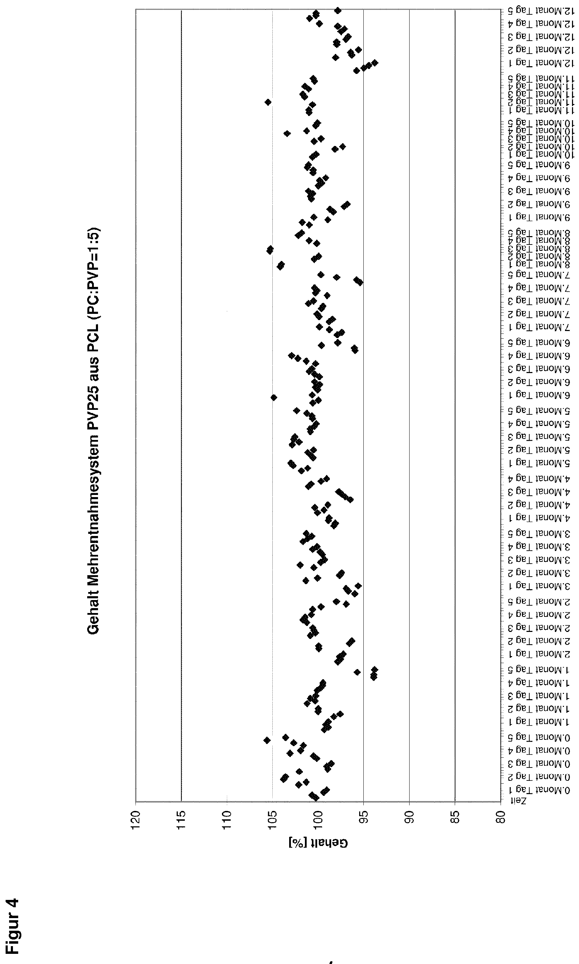

**Fig. 4****:** Stabilität SKA2 (1:5) bei Lagerung im Kühlschrank (Mehrentnahmesystem)

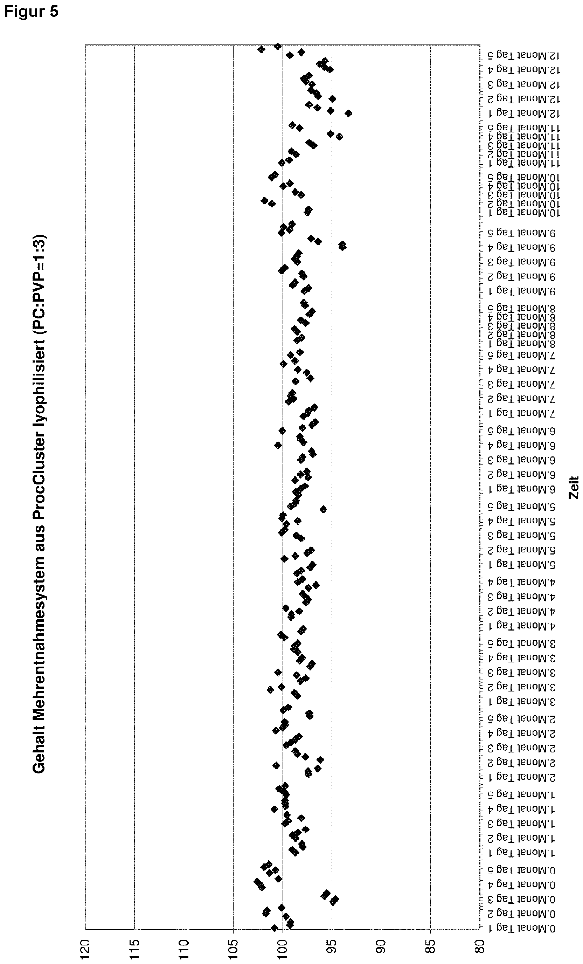

**Fig.** 5: Stabilität SKA3 und SKA4 (mit PVP17 und Galaktose)

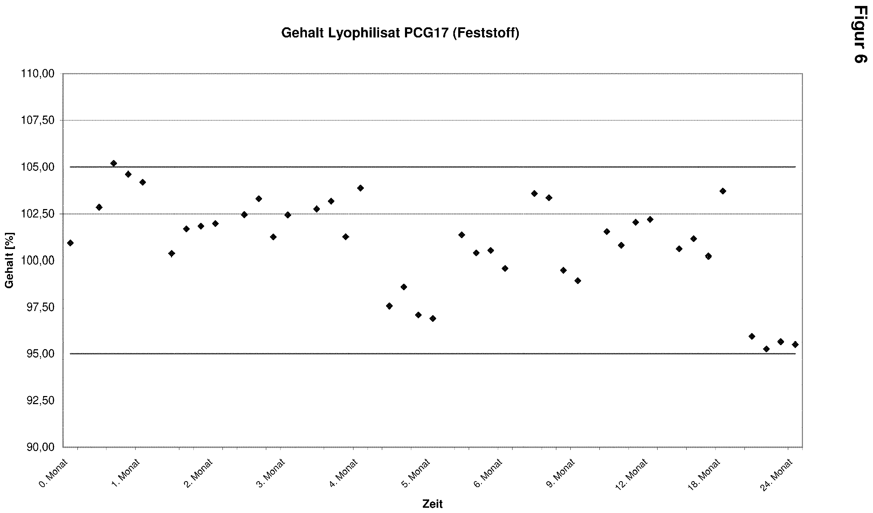

**Fig.** 6: Stabilität SKA1 (1:3) bei Lagerung im Kühlschrank (Mehrentnahmesystem)

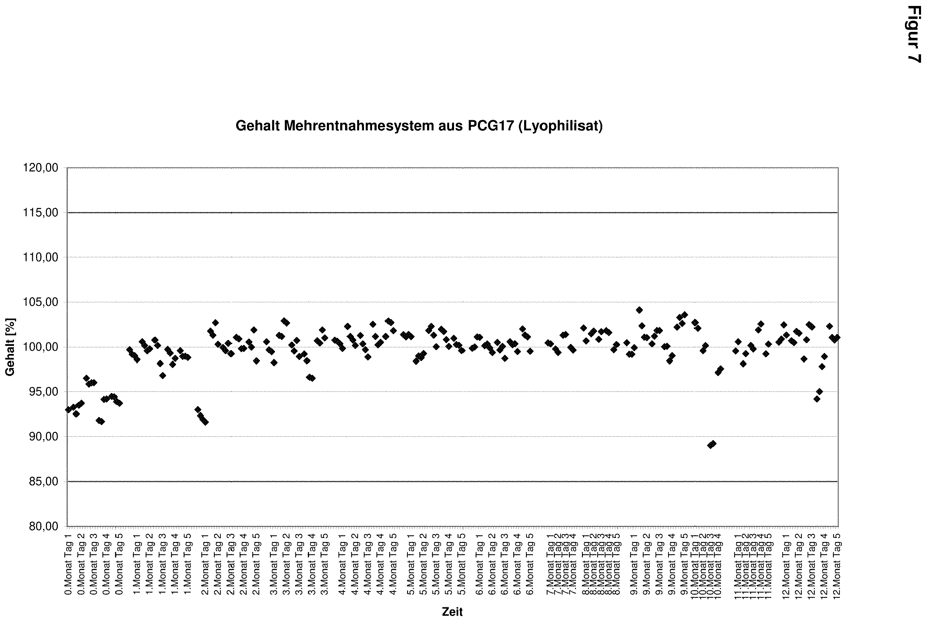

**Fig.** 7: Stabilität SKA3 und SKA4 (mit PVP17 und Galaktose) bei Lagerung im Kühlschrank (Mehrentnahmesystem).
**Fig.** 8: Stabilität Tabletten SKA 4 (ohne weitere Zusätze)

### DETAILIERTE BESCHREIBUNG DER ERFINDUNG

### Substanzen

Die Kohlensäure-Addukte (SKA1), (SKA2), (SKA3) und (SKA4) umfassen jeweils Kohlensäure, mindestens ein Amin (AM) und optional mindestens ein Salz (S).

Unter einem Addukt wird im Rahmen dieser Erfindung ein Molekül verstanden, das durch Zusammenschluss von, bezogen auf ihr Molekulargewicht, kleineren Molekülen als das Addukt, über die Ausbildung von kovalenten oder nicht kovalenten Bindungen, vorzugsweise nicht kovalenten Bindungen gebildet wird. Vorzugsweise umfasst das Addukt als nicht kovalente Bindungen auch ionische Bindungen zwischen zumindest einem Teil der Teilchen, die sich zu dem Addukt zusammengeschlossen haben.

Kohlensäure H₂CO₃, kann in den Kohlensäure-Addukten (SKA1), (SKA2), (SKA3) und (SKA4) sowohl in nicht ionisierter Form, in teilionisierter Form oder auch vollständig ionisiert vorliegen. Diese unterschiedlichen lonisierungsgrade der Kohlensäure können in dem Kohlensäure-Addukt auch nebeneinander vorliegen. Die Ionisierung der Kohlensäure kann durch Übertragung eines oder beider Protonen der Kohlensäure auf ein anderes Molekül, vorzugsweise von mindestens einem Proton auf das Amin (AM) erfolgen.

Unter dem mindestens einem Amin (AM) wird ein Molekül verstanden, dass mindestens eine Aminogruppe, vorzugsweise eine Aminogruppe aufweist. Die Aminogruppe kann eine primäre, sekundäre oder tertiäre Aminogruppe sein.

Die mindestens eine Aminogruppe des Amins (AM) kann in dem Kohlensäure-Addukt (KA), (SKA1), (SKA2), (SKA3), oder (SKA4) sowohl in neutraler Form als auch in protonierter Form vorliegen. Wenn das Amin (AM) über mehr als eine Aminogruppe verfügt, können alle Aminogruppen in neutraler Form oder alle Aminogruppen in protonierter Form oder ein Teil der Aminogruppen in neutraler Form und ein Teil der Aminogruppen in protonierter Form vorliegen. Vorzugsweise liegt mindestens eine Aminogruppe in protonierter Form vor.

Vorzugsweise umfasst das Kohlensäure-Addukt mindestens ein Amin (AM) gemäß einer der nachstehenden Formeln (I) und/oder (II).

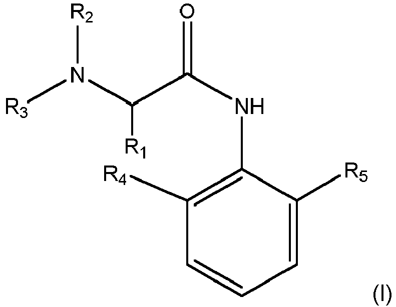

Wobei in Formel (I)
R₁ ist H, C₁₋₁₀ Alkyl, C₂₋₁₀ Alkenyl, Aryl oder Heteroaryl, vorzugsweise H oder C₁₋₁₀ Alkyl, mehr bevorzugt H oder C₁₋₄ Alkyl;
R₂ ist H, C₁₋₁₀ Alkyl, C₂₋₁₀ Alkenyl, Aryl oder Heteroaryl, vorzugsweise H oder C₁₋₁₀ Alkyl, mehr bevorzugt H oder C₁₋₄ Alkyl;
R₃ ist H, C₁₋₁₀ Alkyl, C₂₋₁₀ Alkenyl, Aryl oder Heteroaryl, vorzugsweise H oder C₁₋₁₀ Alkyl, mehr bevorzugt H oder C₁₋₄ Alkyl;
wobei optional R₁ und R₃ miteinander verbunden sein können und zusammen mit dem Stickstoffatom an das R₃ gebunden ist und dem Kohlenstoffatom an das R₁ gebunden ist, einen gesättigten oder ungesättigten, vorzugsweise einen gesättigten Ring bilden können. Vorzugsweise ist der Ring 4, 5 oder 6 gliedrig, mehr bevorzugt 5 oder 6 gliedrig;
R₄ ist H, Halogen, C₁₋₁₀ Alkyl, C₂₋₁₀ Alkenyl, Aryl, Heteroaryl, vorzugsweise H oder Methyl;
R₅ ist H, Halogen, C₁₋₁₀ Alkyl, C₂₋₁₀ Alkenyl, Aryl oder Heteroaryl, vorzugsweise H, Methyl oder Halogen; 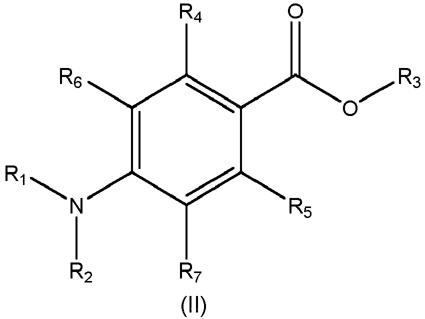 wobei in Formel (II)
R₁ ist H, C₁₋₁₀ Alkyl, C₂₋₁₀ Alkenyl, Aryl oder Heteroaryl, vorzugsweise H oder C₁₋₁₀ Alkyl, mehr bevorzugt H;
R₂ ist H, C₁₋₁₀ Alkyl, C₂₋₁₀ Alkenyl, Aryl oder Heteroaryl, vorzugsweise H oder C₁₋₁₀ Alkyl, mehr bevorzugt H;
R₃ ist H, C₁₋₁₀ Alkyl, C₂₋₁₀ Alkenyl, Aryl, Heteroaryl oder -(CH₂)ₙNR₈R₉, vorzugsweise - (CH₂)ₙNR₈R₉;
n ist 1 bis 5, vorzugsweise 1 bis 3, mehr bevorzugt 1 bis 2;
R₈ ist C₁₋₁₀ Alkyl, vorzugsweise C₁₋₂ Alkyl;
R₉ ist C₁₋₁₀ Alkyl, vorzugsweise C₁₋₂ Alkyl;
R₄ ist H, Halogen, C₁₋₁₀ Alkyl, C₂₋₁₀ Alkenyl, Aryl, Heteroaryl oder -O-C₁₋₁₀ Alkyl, vorzugsweise H, Halogen, C₁₋₁₀ Alkyl oder -O-C₁₋₁₀ Alkyl, mehr bevorzugt H oder Halogen;
R₅ ist H, Halogen, C₁₋₁₀ Alkyl, C₂₋₁₀ Alkenyl, Aryl, Heteroaryl oder -O-C₁₋₁₀ Alkyl, vorzugsweise H, Halogen, C₁₋₁₀Alkyl oder -O-C₁₋₁₀Alkyl, mehr bevorzugt H oder Halogen;
R₆ ist H, Halogen, C₁₋₁₀ Alkyl, C₂₋₁₀ Alkenyl, Aryl, Heteroaryl oder -O-C₁₋₁₀ Alkyl, vorzugsweise H, Halogen, C₁₋₁₀Alkyl oder -O-C₁₋₁₀Alkyl, mehr bevorzugt H oder -O-C₁₋₁₀ Alkyl;
R₇ ist H, Halogen, C₁₋₁₀ Alkyl, C₂₋₁₀ Alkenyl, Aryl, Heteroaryl oder -O-C₁₋₁₀ Alkyl, vorzugsweise H, Halogen, C₁₋₁₀Alkyl oder -O-C₁₋₁₀Alkyl, mehr bevorzugt H oder -O-C₁₋₁₀ Alkyl.

In der vorliegenden Erfindung bedeuten Definitionen wie C₁₋₁₀ Alkyl, wie beispielsweise für den Rest R₁ von Formel (I) definiert, das dieser Substituent (Rest) ein gesättigter Alkylrest mit einer Kohlenstoffanzahl von 1 bis 10 ist. Der Alkylrest kann sowohl linear als auch verzweigt sowie gegebenenfalls zyklisch sein. Alkylreste, die sowohl eine zyklische als auch eine lineare Komponente aufweisen, fallen ebenfalls unter diese Definition. Gleiches gilt für andere Alkylreste wie beispielsweise ein C₁₋₂ Alkylrest. Gegebenenfalls können Alkylreste auch mit funktionellen Gruppen wie Amino, Hydroxy, Halogen, Aryl oder Heteroaryl einfach oder mehrfach substituiert sein. Soweit nicht anders angegeben, weisen Alkylreste vorzugsweise keine funktionellen Gruppen als Substituenten auf. Beispiele für Alkylreste sind Methyl, Ethyl, n-Propyl, n-Butyl, n-Pentyl, n-Hexyl, n-Heptyl, n-Octyl, n-Nonyl, iso-Propyl ( auch 2-Propyl oder 1-Methylethyl genannt), iso-Butyl, tert-Butyl, sec-Pentyl, neo-Pentyl, 1,2-Dimethylpropyl, iso-Amyl, iso-Hexyl, iso-Heptyl.

In der vorliegenden Erfindung bedeuten Definitionen wie C₂₋₁₀ Alkenyl, wie beispielsweise für den Rest R₁ von Formel (I) definiert, das dieser Substituent (Rest) ein Alkenylrest mit einer Kohlenstoffanzahl von 2 bis 10 ist, der mindestens eine ungesättigte Kohlenstoff-Kohlenstoffbindung aufweist. Der Alkenylrest kann sowohl linear als auch verzweigt sowie gegebenenfalls zyklisch sein. Alkenylreste, die sowohl eine zyklische als auch eine lineare Komponente aufweisen, fallen ebenfalls unter diese Definition. Gleiches gilt für andere Alkenylreste wie beispielsweise ein C₂₋₄ Alkenylrest. Gegebenenfalls können Alkenylreste auch mit funktionellen Gruppen wie Amino, Hydroxy, Halogen, Aryl-oder Heteroaryl einfach oder mehrfach substituiert sein. Soweit nicht anders angegeben, weisen Alkenylreste vorzugsweise keine funktionellen Gruppen als Substituenten auf. Beispiele für Alkenylreste sind Vinyl, 1-Propenyl, 2-Propenyl, 1-Butenyl, 2-Butenyl, 3-Butenyl, 1-Pentenyl, 2-Pentenyl, 3-Pentenyl, 4-Pentenyl, 1-Hexenyl, 2-Hexenyl, 3-Hexenyl, 4-Hexenyl, 5-Hexenyl, 1-Heptenyl, 3-Heptenyl, 4-Heptenyl, 1-Octenyl, 3-Octenyl, 5-Octenyl, 1-Nonenyl, 2,-Nonenyl.

In der vorliegenden Erfindung bedeutet Definitionen wie C₂₋₁₀ Alkenyl, wie beispielsweise für den Rest R₁ von Formel (I) definiert, das dieser Substituent (Rest) ein Alkenylrest mit einer Kohlenstoffanzahl von 2 bis 10 ist, der mindestens eine ungesättigte Kohlenstoff-Kohlenstoffbindung aufweist. Der Alkenylrest kann sowohl linear als auch verzweigt sowie gegebenenfalls zyklisch sein. Alkenylreste, die sowohl eine zyklische als auch eine lineare Komponente aufweisen, fallen ebenfalls unter diese Definition. Gleiches gilt für andere Alkenylreste wie beispielsweise ein C₂₋₄ Alkenylrest. Gegebenenfalls können Alkenylreste auch mit funktionellen Gruppen wie Amino, Hydroxy, Halogen, Aryl-oder Heteroaryl einfach oder mehrfach substituiert sein. Soweit nicht anders angegeben, weisen Alkenylreste vorzugsweise keine funktionellen Gruppen als Substituenten auf. Beispiele für Alkenylreste sind Vinyl, 1-Propenyl, 2-Propenyl, 1-Butenyl, 2-Butenyl, 3-Butenyl, 1-Pentenyl, 2-Pentenyl, 3-Pentenyl, 4-Pentenyl, 1-Hexenyl, 2-Hexenyl, 3-Hexenyl, 4-Hexenyl, 5-Hexenyl, 1-Heptenyl, 3-Heptenyl, 4-Heptenyl, 1-Octenyl, 3-Octenyl, 5-Octenyl, 1-Nonenyl, 2,-Nonenyl.

In der vorliegenden Erfindung bedeutet die Definition Aryl, dass es sich um einen aromatischen oder heteroaromatischen Rest handelt. Ein aromatischer Rest ist ein aromatischer cyclischer Kohlenwasserstoff, der aus einem Ring oder einem Ringsystem aus mehreren kondensierten Ringen bestehen kann. Der aromatische Rest kann beispielsweise monocyclisch, bicyclisch oder tricyclisch sein. Vorzugsweise bildet der monocyclische aromatische Rest einen 5- oder 6-gliedrigen Ring. Vorzugsweise bildet der bicyclische aromatische Ring einen 9- oder 10-gliedrigen Ring. Vorzugsweise bildet der tricyclische aromatische Ring einen 13- oder 14-gliedrigen Ring. Die Arylgruppe enthält vorzugsweise 3 bis 14, mehr bevorzugt 4 bis 6 Kohlenstoffatome. Gegebenenfalls können Arylreste auch mit funktionellen Gruppen wie Alkyl, Alkenyl, Amino, Hydroxy, Halogen, Aryl oder Heteroaryl einfach oder mehrfach substituiert sein. Beispiele für aromatische Reste sind Phenyl, und Naphtyl.

In der vorliegenden Erfindung bedeutet die Definition Heteroaryl, dass es sich um einen heteroaromatischen Rest handelt. Heteroaromatischer Ring bedeutet, dass in einem aromatischen Rest, wie vorstehend definiert, dessen Ringsystem durch Kohlenstoffatome gebildet wird, ein oder mehrere dieser Kohlenstoffatome durch Heteroatome wie O, N oder S ersetzt sind. Beispiele für heteroaromatische Reste, die in der vorliegenden Erfindung unter die Definition Aryl fallen, sind Furanyl, Thienyl, Oxazolyl, Pyrazolyl, Pyridyl und Indolyl.

In der vorliegenden Erfindung bedeutet die Definition Halogen, wie beispielsweise vorstehend für den Rest R₄ für Formel (I) definiert, dass es sich um einen Chlor-, Brom-, lod- oder Fluorsubstituenten handelt. Vorzugsweise handelt es sich um einen Chlor- oder Fluorsubstituenten.

Mehr bevorzugt umfasst das Kohlensäure-Addukt (KA), (SKA1), (SKA2), (SKA3), oder (SKA4) mindestens ein Amin (AM) ausgewählt aus der Gruppe 4-Aminobenzoesäure-2-(*N*,*N-*diethylamino-)ethylester (Procain), 4-Aminobenzoesäureethylester (Benzocain), 2-(Diethylamino)ethyl-4-amino-2-chlorbenzoat (Chlorprocain), 4-Amino-3-butoxybenzoesäure-2-diethylaminoethylester (Oxybuprocain), (2-(Dimethylamino)ethyl)-4-(butylamino)benzoat (Tetracain), N-[3-(4-Phenoxymethylphenyl)-propyl]morpholin (Fomocain), 2-Diethylamino-N-(2,6-dimethylphenyl)acetamid (Lidocain), (RS)-N-(2,6-Dimethylphenyl)-1-methylpiperidin-2-carboxamid (Mepivacain), (RS)-N-(2-Methylphenyl)-2-(propylamino)-propanamid (Prilocain), (RS)-4-Methyl-3-[2-(propylamino)-propanamido]thiophen-2-carbonsäuremethylester (Articain), (±)-1-Butyl-N-(2,6-dimethylphenyl)-2-piperidincarboxamid (Bupivacain), (S)-1-Propyl-2',6'-dimethyl-2-piperidylcarboxyanilid (Ropivacain), 2-(Ethylpropylamino)-2',6'-butyroxylidid (Etidocain) und 1-(4-Butoxyphenyl)-3-piperidin-1-ylpropan-1-on (Dyclonin).

Noch mehr bevorzugt umfasst das Kohlensäure-Addukt (KA), (SKA1), (SKA2), (SKA3), oder (SKA4) mindestens ein Amin ausgewählt aus der Gruppe 4-Aminobenzoesäure-2-(*N*,*N-*diethylamino-)ethylester (Procain), 2-Diethylamino-N-(2,6-dimethylphenyl)acetamid (Lidocain) und (2-(Dimethylamino)ethyl)-4-(butylamino)benzoat (Tetracain).

Besonders bevorzugt umfasst das Kohlensäure-Addukt (KA), (SKA1), (SKA2), (SKA3), oder (SKA4) mindestens ein Amin (AM) ausgewählt aus der Gruppe 4-Aminobenzoesäure-2-(*N*,*N*-diethylamino-)ethylester (Procain) und 2-Diethylamino-N-(2,6-dimethylphenyl)acetamid (Lidocain).

Ganz besonders bevorzugt umfasst das Kohlensäure-Addukt (KA), (SKA1), (SKA2), (SKA3), oder (SKA4) 4-Aminobenzoesäure-2-(N,N-diethylamino-)ethylester (Procain) als Amin (AM).

Optional umfasst das Kohlensäure-Addukt (SKA1), (SKA2), (SKA3), oder (SKA4) mindestens ein Salz (S). Vorzugsweise umfasst das Kohlensäure-Addukt (SKA1), (SKA2), (SKA3), oder (SKA4) mindestens ein Salz (S). Vorzugsweise umfasst das Kohlensäure-Addukt (AM) mindestens ein Salz (S), wenn das Amin (AM) Procain oder Lidocain ist.

Bei dem mindestens einen Salz (S) handelt es sich vorzugsweise um ein Salz, das aus mindestens einem Kation ausgewählt aus Na⁺, K⁺, Li⁺, Mg²⁺, Zn²⁺, Fe²⁺, Fe³⁺ und Mn²⁺ und mindestens einem Anion ausgewählt aus Cl⁻, Br, J⁻, F⁻, SO₄²⁻, SO₃²⁻, HSO₄⁻ HSO₃⁻, ⁻HCO₃⁻, CO₃²⁻, PO₄³⁻, HPO₄²⁻, H₂PO₄⁻, SiO₄⁴⁻, AlO₂⁻, SiO₃⁻ und /oder [AlO₂)₁₂(SiO₂)₂]²⁻ zusammengesetzt ist. Mehr bevorzugt ist das Kation ausgewählt aus Na⁺ und das Anion ausgewählt aus Cl⁻ oder Br.' Das Salz kann beispielsweise, durch eine Säure-Base-Reaktion der Base (BA), bei Durchführung von Schritt b) mit der an das Amin (AM) addierten Säure gebildet werden, falls ein Säureaddtionssalz des Amins (AM) eingesetzt wird. Das Salz (S) kann auch direkt in einem der Schritte a), b) und/oder c) zugesetzt werden. Der direkte Zusatz des Salzes (S) ist bevorzugt, falls das Amin (AM) nicht in der Salzform eingesetzt und/oder der Schritt b) nicht durchgeführt wird.

Das Kohlensäure-Addukt (KA), (SKA1), (SKA2), (SKA3), oder (SKA4) ist herstellbar nach einem Verfahren umfassend unter anderem. die Schritte a), optional b), c), optional d) und optional e).

In Schritt a) wird eine Lösung (A) bereitgestellt, die mindestens ein Lösungsmittel und in dem mindestens einen Lösungsmittel gelöstes CO₂ umfasst.

Die Lösung (A) umfasst mindestens ein Lösungsmittel und CO₂ in gelöster Form. Unter CO₂ wird im Rahmen dieser Erfindung Kohlendioxid verstanden. Unter CO₂ in gelöster Form werden im Rahmen dieser Erfindung alle Formen des CO₂ verstanden, die dieses beim Lösen eingeht. So ist beispielweise für wässrige Lösungen bekannt, dass das gelöste CO₂ in der Lösung unter anderem im Gleichgewicht als CO₂, als Kohlensäure, als einfach oder zweifach deprotonierte Kohlensäure, das heißt als Hydrogencarbonat oder Carbonat vorliegen kann.

Die Lösung (A) wird erhalten, in dem CO₂ in das mindestens eine Lösungsmittel eingebracht wird. Das CO₂ kann in allen dem Fachmann bekannten und geeigneten Formen und in das Lösungsmittel eingebracht werden. Vorzugsweise wird gasförmiges oder gefrorenes CO₂ in Form von Trockeneis, mehr bevorzugt gasförmiges CO₂ in das Lösungsmittel eingebracht. Das CO₂ kann auch unter Druck eingebracht werden, insbesondere wenn gasförmiges CO₂ in die Lösung eingebracht wird. Einbringen unter Druck bedeutet in diesem Zusammenhang, dass ein Druck größer dem Atmosphärendruck, vorzugsweise größer 1,01325 bar, verwendet wird. Zu diesem Zweck kann das Einbringen des CO₂ in das Lösungsmittel in einem Behälter erfolgen, der das Lösungsmittel derartig von der Umgebung isoliert, dass in dem Behälter ein Druck erzeugt werden kann, insbesondere über das Zuleiten des CO₂, der über dem atmosphärischen Druck, vorzugsweise über 1,01325 bar liegt. Das Einbringen des CO₂ in die Lösung, insbesondere in gasförmiger Form, kann in einem Schritt oder in Intervallen erfolgen.

Der Fachmann kann in Schritt a) jedes geeignete Lösungsmittel einsetzen. Vorzugsweise wird als Lösungsmittel ein polar-protisches Lösungsmittel eingesetzt, mehr bevorzugt ist das Lösungsmittel Wasser. Das Lösungsmittel kann je nach beabsichtigtem Verwendungszweck des Kohlensäure-Addukts (KA), (SKA1), (SKA2), (SKA3), oder (SKA4) in verschiedenen Reinheitsgraden eingesetzt werden. Beispielsweise kann Wasser mit dem Reinheitsgrad "Aqua ad iniectabilia" verwendet werden, falls das Kohlensäure-Addukt (SKA1), (SKA2), (SKA3) oder (SKA4) für pharmazeutisch-medizinische Zwecke eingesetzt werden soll.

Schritt a) kann den Teilschritt a1) umfassen, wobei das Lösungsmittel, vorzugsweise vor dem Einbringen des CO₂, auf 3 bis 8 °C, vorzugsweise auf 5 °C gekühlt wird. Das Kühlen kann mittels allen dem Fachmann bekannten und als geeignet identifizierten Methoden erfolgen. Beispielsweise kann das Kühlen durch Verwahren des Lösungsmittels für eine ausreichend lange Zeit in einem Kühlschrank erfolgen, bis das Lösungsmittel die Zieltemperatur aufweist. Ebenso kann beispielsweise eine externe Kühlung verwendet werden.

Schritt a) kann den Teilschritt a2) umfassen, wobei das CO₂ in das Lösungsmittel eingebracht wird, vorzugsweise bis zum Erreichen einer Sättigungskonzentration von 3 bis 10 g/l, mehr bevorzugt bis zu einer Sättigungskonzentration von 4,5 bis 7,5 g/l bezogen auf das Gesamtvolumen der Lösung. Vorzugsweise beträgt der pH-Wert der Lösung nach der Sättigung mit CO₂ ≤ 3,0 bis ≤ 6,0, noch mehr bevorzugt ≤ 4,3 bis ≤ 4,8. Vorzugsweise wird das CO₂ in Teilschritt a2) unter Druck gelöst, wobei der Druck 1,5 bis 10 bar, mehr bevorzugt 1,9 bis 7 bar, noch mehr bevorzugt 2 bis 5 bar beträgt.

Schritt a) kann den Teilschritt a3) umfassen, wobei die vorzugsweise in Teilschritt a2) erhaltene Lösung (A) bei 1 bis 10 °C vorzugsweise für mindestens 30 min, mehr bevorzugt für mindestens 50 min, noch mehr bevorzugt für mindestens 60 min; bis höchstens 5d (120h) gelagert wird. Vorzugsweise wird die vorzugsweise in Teilschritt a2) erhaltene Lösung (A) bei 3 bis 8 °C für mindestens 30 min, mehr bevorzugt für mindestens 50 min, noch mehr bevorzugt für mindestens 60 min; bis höchstens 5d (120h) gelagert.

Vorzugsweise umfasst Schritt a) alle Teilschritte a1), a2) und a3).

Vorzugsweise werden die Teilschritte a1), a2) und a3) in der Reihenfolge a2) folgt auf a1) und a3) folgt auf a2) durchgeführt.

Optional kann der Schritt b) durchgeführt werden, wobei die Base (BA), die nicht dem Amin (AM) entspricht, in der Lösung (A), unter Erhalt der Lösung (A1) aufgelöst wird. Vorzugsweise ist die Base (BA) ein Hydrogencarbonat oder ein Carbonat, mehr bevorzugt ein Hydrogencarbonat, noch mehr bevorzugt Natriumhydrogencarbonat.

In Schritt c) wird das mindestens eine Amin (AM) in der Lösung (A) oder (A1) unter Erhalt der Lösung (B) gelöst.

Das mindestens eine Amin (AM), wie vorstehend definiert, kann in Schritt c) sowohl in neutraler Form als auch in Form eines Salzes eingesetzt werden. Optional kann das mindestens eine Amin (AM) auch als Mischung der neutralen Form des Amins (AM) mit der Salzform des Amins (AM) eingesetzt werden. Daher kann das mindestens eine Amin (AM), das neutrale Amin (AM) und/oder die Salzform des mindestens einen Amins (AM) umfassen. Vorzugsweise handelt es sich bei der Salzform des mindestens einen Amins (AM) um ein Säureadditionssalz, vorzugsweise ist das Säureadditionsalz ein Hydrochlorid, Hydrobromid, Hydroiodid, Hydrogensulfat, Hydrogensulfit, Hydrogenphosphat, Hydromesylat, Hydrotosylat, Hydroacetat, Hydroformiat, Hydropropanoat, Hydromalonat, Hydrosuccinat, Hydrofumarat, Hydroxalat, Hydrotartrat, Hydrocitrat, Hydromaleat, mehr bevorzugt ein Hydrochlorid oder Hydrobromid, noch mehr bevorzugt ein Hydrochlorid des mindestens einen Amins (AM).

Vorzugsweise beträgt die Konzentration des Amins (AM) in Lösung (B) 0,01 bis 0,25 g/ml, vorzugsweise 0,03 bis 0,20 g/ml, mehr bevorzugt 0,08 bis 0,15 g/ml.

Schritt c) kann den Teilschritt c2) umfassen, wobei das mindestens eine Amin (AM) in Lösung (A), oder bei Durchführung von Schritt b), in Lösung (A1) unter Erhalt der Lösung (B) aufgelöst wird.

In einer möglichen Ausführungsform beträgt das Verhältnis des Amins (AM) zur Base (BA), bei Durchführung von Schritt b), in Lösung (B) 2:1 bis 5:1, mehr bevorzugt 3:1 bis 4:1, noch mehr bevorzugt 3,23:1 bis 3,26:1 [g/g]].

In einer weiteren möglichen Ausführungsform beträgt das molare Verhältnis des Amins (AM) zu den Basenequivalenten der Base (BA), bei Durchführung von Schritt b), in Lösung (B) 0,8:1 bis 1,5:1, vorzugsweise 1,2:1, mehr bevorzugt 1:1. Basenequivalente bedeutet in diesem Zusammenhang, dass bei Verwendung einer einwertigen Base, wie zum Beispiel NaHCO₃ das molare Verhältnis der Base (BA) zum Amin (AM) dem vorstehend angegebenen Verhältnis entspricht. Bei Verwendung einer zweiwertigen Base (BA), wie zum Beispiel Na₂CO₃, ist bezogen auf die Stoffmenge in Mol der Base (BA) relativ zur Verwendung einer einwertigen Base nur die Hälfte der Basenmenge erforderlich um die gleiche Menge an Basenequivalenten einzubringen. So werden zum Beispiel bei einem Verhältnis von 1:1, bei Verwendung von 10 mmol Amin (AM), 10 mmol NaHCO₃ benötigt, aber nur 5 mmol Na₂CO₃.

In einer weiteren Ausführungsform wird Schritt b) ausgeführt und in Teilschritt c1) das Amin (AM) in Form des Säureadditionssalzes zugesetzt, wobei das Amin (AM) mit der an ihm gebundenen Säure, in einer derartigen Menge zugesetzt wird, dass die an das Amin (AM) gebundene Säure, die Base (BA) soweit zu neutralisieren vermag, dass die Lösung (B) einen pH-Wert von 6 bis 8 annimmt.

Schritt c) kann den Teilschritt c2) umfassen, wobei Lösung (A) zur Lösung (B) unter Erhalt der Lösung (B1) gegeben wird.

Vorzugsweise beträgt die Konzentration des Amins (AM) in Lösung (B1) 0,01 bis 0,25 g/ml, vorzugsweise 0,03 bis 0,20 g/ml, mehr bevorzugt 0,08 bis 0,15 g/ml.

Schritt c) kann den Teilschritt c3) umfassen, wobei die Lösung (B) oder bei Durchführung von Teilschritt c2), die Lösung (B1) mit CO₂ angereichert wird. Vorzugsweise wird die Lösung (B) mit 2,5 g/l bis 9 g/l, mehr bevorzugt mit 5 bis 7,5 g/l CO₂ angereichert.

Schritt c) kann den Teilschritt c4) umfassen, wobei die Lösung (B) oder, bei Durchführung von Teilschritt c2), die Lösung (B1) bei 1 bis 10°C, vorzugsweise 3 bis 8 °C, für mindestens 1 h vorzugsweise 24 h bis 120 h, noch mehr bevorzugt 24 bis 72 h gelagert wird.

Schritt c) kann den Teilschritt c5) umfassen, wobei die Lösung (B) oder bei Durchführung von Teilschritt b2) die Lösung (B1) mit CO₂ auf eine Gesamtkonzentration von mindestens 6 g/l, vorzugsweise mindestens 10 g/l, mehr bevorzugt mindestens 12 g/l, noch mehr bevorzugt mindestens 14 g/l und ganz besonders bevorzugt mindestens 15 g/l angereichert wird. Vorzugsweise werden in Teilschritt c5) weitere 0,4 bis 4,7 g/l, mehr bevorzugt 1 bis 3,5 g/l CO₂ bis zum Erreichen der benötigten Gesamtkonzentration in die Lösung (B) oder (B1) eingebracht bzw. gelöst.

Der Begriff "Gesamtkonzentration" bezieht sich hier auf die Gesamtkonzentration an gelöstem CO₂ in der Lösung (B) oder (B1), einschließlich dem CO₂, das im Kohlensäure-Addukt (KA) gebunden ist. Die Gesamtkonzentration ergibt sich additiv aus der Gewichtszunahme der Lösung durch das zugeführte CO₂ in allen vorangegangenen Anreicherungsschritten a2) und/oder c3), soweit ausgeführt, und c5), ohne Berücksichtigung von CO₂, das optional in Form von Hydrogencarbonat oder Carbonat als Base (BA) der Lösung zugesetzt wird.

Das Anreichern der Lösung (B) oder der Lösung (B1) in Teilschritt c5) mit CO₂ auf die benötigte Gesamtkonzentration kann bei einem Druck von 2,5 bis 10 bar, vorzugsweise von 4 bis 10 bar, mehr bevorzugt von 5 bis 10 bar, noch mehr bevorzugt bei 6 bis 10 bar, ganz besonders bevorzugt bei 6,5 bis 10 bar durchgeführt werden. Vorzugsweise weist die Lösung (B) oder (B1), beim Anreichern mit CO₂ in Teilschritt c5) eine Temperatur von 3 bis 8 °C, mehr bevorzugt von 5°C auf.

Die Anreichung der Lösung (B) oder (B1) in den Teilschritten c3) und c5) kann auf dieselbe Weise erfolgen, wie für Schritt a) beschrieben.

Vorzugsweise beträgt der pH-Wert der Lösung (B) oder, bei Durchführung des Teilschritts c2), der Lösung (B1) nach der Durchführung von Schritt c5) ≤ 7,0.

Vorzugsweise umfasst Schritt c) alle Teilschritte c1), c2), c3), c4) und c5).

Vorzugsweise werden die Teilschritte c1), c2), c3), c4) und c5) in der Reihenfolge c2) folgt auf c1), c3) folgt auf c2), c4) folgt auf c3), c5) folgt auf c4) durchgeführt.

Die Herstellung des stabilisierten Kohlensäure-Addukts (SKA1) und (SKA4) umfasst den Schritt d). In Schritt d) wird die nach Abschluss des Schrittes c) erhaltene Lösung eingefroren. Vorzugsweise wird in Schritt d) die Lösung B) oder nach Durchführung des Teilschrittes c2), die Lösung (B1) eingefroren.

Die Lösung, vorzugsweise Lösung (B) oder (B1), die dem Schritt d) unterzogen wird, weist bei der Herstellung von (SKA1) einen Gehalt an CO₂ von mindestens 6 g/l, vorzugsweise mindestens 7 g/l, mehr bevorzugt mindestens 8 g/l, besonders bevorzugt 8,1 g/l bis 9,5 g/l auf.

Vorzugsweise wird die nach Abschluss des Schrittes c) erhaltene Lösung, vorzugsweise Lösung (B) oder (B1), in Schritt d) bei -100 °C bis -20 °C, mehr bevorzugt bei -90 °C bis -30 °C, noch mehr bevorzugt bei -80 bis -40 °C und ganz besonders bevorzugt bei -70 bis -50 °C in Schritt d) eingefroren.

Das Einfrieren der nach Abschluss des Schrittes c) erhaltenen Lösung, vorzugsweise Lösung (B) oder (B1), in Schritt d), kann grundsätzlich nach allen dem Fachmann bekannten und als geeignet identifizierten Methoden erfolgen. Beispielsweise kann das Einfrieren durch das Überführen der in Schritt c) erhaltenen Lösung in ein geeignetes Gefäß, erfolgen, dass in ein Kühlmedium eintaucht. Vorzugsweise hat das Gefäß eine Kolbenform. Vorzugsweise taucht das Gefäß, in dem sich die in Schritt c) erhaltene Lösung befindet in einem Winkel von 40 ° in das Kühlmedium ein. Das Kühlmedium kann beispielsweise aus einem Lösungsmittel wie Methanol, Ethanol oder Aceton bestehen, das durch Zugabe von Trockeneis, oder geeignete Kühlapparaturen, wie Kryostaten auf die gewünschte Temperatur gebracht wird.

Vorzugsweise erfolgt das Einfrieren in Schritt d) bei Atmosphärendruck, mehr bevorzugt bei 1,01325 bar.

Vorzugsweise wird die nach Abschluss des Schrittes c) erhaltene Lösung, vorzugsweise Lösung (B) oder (B1), in Schritt d) innerhalb von 0,3 bis 60 Minuten, mehr bevorzugt innerhalb von 1 bis 30 Minuten, noch mehr bevorzugt innerhalb von 1,1 bis 10 Minuten, besonders bevorzugt innerhalb von 1,5 bis 5 Minuten eingefroren.

Die nach Abschluss des Schrittes c) erhaltene Lösung, vorzugsweise Lösung (B) oder (B1), wird in Schritt d) vorzugsweise mit einer Kühlrate von 10 bis 100 K/min, mehr bevorzugt mit 20 bis 80 K/min, noch mehr bevorzugt mit 30 bis 70 K/min und besonders bevorzugt mit 40 bis 60 K/min eingefroren.

Vorzugsweise wird das Gefäß in dem sich die nach Abschluss des Schrittes c) erhaltene Lösung, vorzugsweise Lösung (B) oder (B1), in Schritt d) während des Einfriervorgangs befindet, im Kühlmedium, mit 10 bis 1000 rpm, mehr bevorzugt mit 50 bis 600 rpm, noch mehr bevorzugt mit 100 bis 400 rpm und besonders bevorzugt mit 200 bis 300 rpm rotiert.

Das Einfrieren in Schritt d) kann nach dem Shell-Freeze-Verfahren erfolgen.

Die Herstellung des stabilisierten Kohlensäure-Addukts (SKA1) und (SKA4) umfasst den Schritt e). In Schritt e) wird die in Schritt d) eingefrorene Lösung, vorzugsweise Lösung (B) oder (B1), bei -100 bis 0 °C für nicht länger als 4 Tage gelagert.

Vorzugsweise wird die in Schritt d) eingefrorene Lösung in Schritt e), vorzugsweise Lösung (B) oder (B1), für 1,5 bis 4 Tage, mehr bevorzugt für 2,5 bis 4 Tage gelagert.

Vorzugsweise wird die in Schritt d) eingefrorene Lösung in Schritt e), vorzugsweise Lösung (B) oder (B1) bei -50 bis 0°C, mehr bevorzugt bei -30 bis -5 °C, noch mehr bevorzugt bei -25 bis -10 °C, besonders bevorzugt bei -20 bis -15 °C gelagert.

Die Lagerung in Schritt e) kann bei der definierten Temperatur grundsätzlich in jeder dem Fachmann bekannten Kühleinrichtung erfolgen. Beispielsweise kann die Lagerung in einem Tiefkühlschrank oder einem Tiefkühlraum durchgeführt werden.

Das Verfahren nach dem das Kohlensäure-Addukt herstellbar ist umfasst den Schritt f). Das Verfahren nach dem das Kohlensäure-Addukt (SKA1) oder (SKA3) herstellbar ist, kann einen weiteren Schritt f) umfassen. Der Schritt f) wird nach Schritt e) oder j) durchgeführt wird. Dabei wird in Schritt f) die in Schritt e) oder j) gelagerte Lösung, vorzugsweise Lösung (B) oder (B1), getrocknet, unter Erhalt von getrocknetem Kohlensäure-Addukt (KA), (SKA1) oder (SKA3).

Vorzugsweise wird in Schritt f) das Wasser aus der in Schritt e) oder j) gelagerten Lösung, vorzugsweise Lösung (B) oder (B1) bis zu einem Restgehalt von < 0,8 Gew.-%, mehr bevorzugt bis zu einem Restgehalt von < 0,1 Gew.-% bezogen auf das Gesamtgewicht des getrockneten Kohlensäure-Adduktes (KA) entfernt.

Vorzugsweise wird in Schritt f) nicht im Kohlensäure-Addukt (SKA1) oder (SKA3) gebundenes CO₂ aus der in Schritt e) oder j) gelagerten Lösung, vorzugsweise (B) oder (B1), bis zu einem Restgehalt von < 0,8 Gew.-%, mehr bevorzugt bis zu einem Restgehalt von < 0,1 Gew.-% bezogen auf das Gesamtgewicht des getrockneten Kohlensäure-Adduktes (SKA1) oder (SKA3) entfernt.

Die Trocknung kann mit allem dem Fachmann bekannten und als geeignet identifizierten Methoden durchgeführt werden. Vorzugsweise erfolgt die Trocknung mittels Gefriertrocknung, auch Lyophilisation genannt. Der Schritt d) stellt im Falle der Anwendung des Gefriertrocknungsverfahrens, den Einfrierschritt und Schritt e) bzw. j) den Reifungsschritt dar. Die Lyophilisation kann auch als Plattenlyophilisation oder Sprühgefriertrocknung durchgeführt werden.

Vorzugsweise beträgt der Druck während des Trocknens in Schritt f) 0,01 bis 30 mbar, vorzugsweise 0,02 bis 20 mbar, mehr bevorzugt 0,03 bis 10 mbar, noch mehr bevorzugt 0,03 bis 0,5 mbar und ganz besonders bevorzugt 0,05 bis 0,1 mbar. Vorzugsweise wird der Druck während des gesamten Trocknungsvorgangs beibehalten. Vorzugsweise wird der vorstehend definierte Druck während des Trocknens innerhalb von 7h, mehr bevorzugt innerhalb von 5h und besonders bevorzugt innerhalb von 4 h ab Evakuierungsbeginn erreicht.

Den Endpunkt der Trocknung kann der Fachmann aus den Temperaturverlaufsaufzeichnungen ermitteln. Vorzugsweise beträgt die Gesamttrocknungszeit in Schritt f) 10 bis 60 h, mehr bevorzugt 30 bis 55 h, besonders bevorzugt 41 bis 52 h. Die Gesamttrocknungszeit ist definiert als die Zeitspanne zwischen dem Abschluss der Lagerung in Schritt e) or j) und der Beendigung der Trocknung in Schritt f).

Vorzugsweise beträgt die Temperatur während der gesamten Trocknung in Schritt f) 0 bis 20 °C, vorzugsweise 4 bis 18 °C, mehr bevorzugt 8 bis 16 °C.

Das Verfahren zur Herstellung des Kohlensäure-Addukts (SKA1) umfasst den Schritt g1), Zugabe von Polyvinylpyrrolidon zu dem in den vorangehenden Schritten erhaltenen Kohlensäure-Addukt (KA) nach Ausführung des Schritts e) unter Erhalt des stabilisierten Kohlensäure-Addukts (SKA1).

In Schritt g1) beträgt das Gewichtsverhältnis Polyvinylpyrrolidon (PVP) zum Kohlensäure-Addukt (KA) vorzugsweise 10:1, mehr bevorzugt 5:1, besonders bevorzugt 3:1 beträgt.

Die Zugabe von Polyvinylpyrrolidon in Schritt g1) kann sowohl mit Polyvinylpyrrolidon als Feststoff oder als Lösung in Wasser erfolgen. Die Konzentration des Polyvinylpyrrolidons in der zugegebenen Lösung wird je nach benötigter Polyvinylpyrrolidinmenge in Schritt g1) eingestellt, abhängig vom Gewichtsverhältnis von Polyvinylpyrrolidon zum Kohlensäure-Addukt (KA). Vorzugsweise beträgt die Konzentration an Polyvinylpyrrolidon in Wasser 0,5 bis 20 Gew.-%, mehr bevorzugt 0,9 bis 18 Gew.-%. Vorzugsweise beträgt die Konzentration an Kohlensäure-Addukt (KA) 0,15 Gew.-% bis 5,7 Gew.-%.

In einer Ausführungsform, vorzugsweise zu Inhalation, beträgt das Gewichtsverhältnis Polyvinylpyrrolidon (PVP) zum Kohlensäure-Addukt (KA) vorzugsweise 3:1, und die Konzentration an Polyvinylpyrrolidon (PVP) 0,9 Gew.-% in Wasser. Vorzugsweise beträgt die Konzentration an Kohlensäure-Addukt (KA) 0,15 Gew.-%.

In einer Ausführungsform, vorzugsweise zu buccalen Anwendung, beträgt das Gewichtsverhältnis Polyvinylpyrrolidon (PVP) zum Kohlensäure-Addukt (KA) vorzugsweise 3:1, und die Konzentration an Polyvinylpyrrolidon (PVP) 17,1 Gew.-% in Wasser. Vorzugsweise beträgt die Konzentration an Kohlensäure-Addukt (KA) 5,7 Gew.-%.

In einer Ausführungsform, vorzugsweise zu parentalen Anwendung, beträgt das Gewichtsverhältnis Polyvinylpyrrolidon (PVP) zum Kohlensäure-Addukt (KA) vorzugsweise 3:1, und die Konzentration an Polyvinylpyrrolidon (PVP) 6 Gew.-%. Vorzugsweise beträgt die Konzentration an Kohlensäure-Addukt (KA) 2 Gew.-%.

Vorzugsweise wird Schritt g1) nach dem auf den Schritt e) folgenden Schritt f) ausgeführt. In dieser Ausführungsform werden das Kohlensäure-Addukt (KA) und das Polyvinylpyrrolidon in Form ihrer Feststoffe, vorzugsweise als Pulver, vermengt unter Erhalt des stabilisierten Kohlensäure-Addukts (SKA1).

In Schritt g1) weist das Polyvinylpyrrolidon (PVP) vorzugsweise ein durchschnittliches Molekulargewicht von 10000 bis 40000 g/mol, mehr bevorzugt von 15000 bis 35000 g/mol, noch mehr bevorzugt von 24000 bis 33000 g/mol, besonders bevorzugt von 17000 g/mol bis 32000 g/mol auf.

Das Verfahren zur Herstellung des Kohlensäure-Addukts (SKA2) umfasst den Schritt g2), Zugabe von Polyvinylpyrrolidon zu der in Schritt c) erhaltenen Lösung (B) unter Erhalt des stabilisierten Kohlensäure-Addukts (SKA2),

In Schritt g2) beträgt das Gewichtsverhältnis Polyvinylpyrrolidon (PVP) zum Kohlensäure-Addukt (KA) vorzugsweise 1:1 bis 20: 1, mehr bevorzugt 4:1 bis 10:1, besonders bevorzugt 5:1 beträgt.

Die Zugabe von Polyvinylpyrrolidon in Schritt g2) kann sowohl mit Polyvinylpyrrolidon als Feststoff oder als Lösung in Wasser erfolgen. Die Konzentration des Polyvinylpyrrolidons in der zugegebenen Lösung wird je nach benötigter Polyvinylpyrrolidinmenge in Schritt g2) eingestellt, abhängig vom Gewichtsverhältnis von Polyvinylpyrrolidon zum Kohlensäure-Addukt (KA)..Vorzugsweise beträgt die Konzentration an Kohlensäure-Addukt (KA) 0,15 Gew.-% bis 5,7 Gew.-% in Wasser. Vorzugsweise beträgt die Konzentration an Polyvinylpyrrolidon in Wasser 0,75 bis 30 Gew.-%, mehr bevorzugt 1,5 bis 28,5 Gew.-%.

Bei Herstellung des stabilisierten Kohlensäure-Addukts (SKA2) beträgt der Gehalt an CO₂ in der Lösung (B), die dem Schritt c) unterzogen wird, mindestens 10 g/l, vorzugsweise mindestens 12 g/l, mehr bevorzugt mindestens 14g/l und noch mehr bevorzugt mindestens 14 to 15 g/l beträgt und das Amin (AM) auch in Form eines Salzes eingesetzt werden kann.

In Schritt g2) weist das Polyvinylpyrrolidon (PVP) vorzugsweise ein durchschnittliches Molekulargewicht von von 15000 bis 35000 g/mol, mehr bevorzugt von 24000 bis 33000 g/mol, besonders bevorzugt von 16000 g/mol bis 27000 g/mol auf.

Das Verfahren zur Herstellung des Kohlensäure-Addukts (SKA3) umfasst den Schritt g3), Zugabe von Polyvinylpyrrolidon zu der in Schritt c) erhaltenen Lösung (B) unter Erhalt der Lösung (C).

Vorzugsweise beträgt in Schritt g3) das Gewichtsverhältnis Polyvinylpyrrolidon (PVP) zum Kohlensäure-Addukt (KA) 6:1 bis 1:0,5, mehr bevorzugt 3:1, besonders bevorzugt 1,5:1.

Vorzugsweise erfolgt die Zugabe des Polyvinylpyrrolidons als Lösung. Mehr bevorzugt erfolgt die Zugabe des Polyvinylpyrrolidons in Wasser als Lösungsmittel. Vorzugsweise beträgt in dieser Lösung das Gewichtsverhältnis (w/w) von Polyvinylpyrrolidon:Wasser 1,1 bis 0,75, mehr bevorzugt 0,9 bis 0,8, besonders bevorzugt 0,85.

Vorzugsweise werden in Schritt g3) ein oder mehrere weitere Additive zugesetzt, ausgewählt aus einem Monosaccharid, mehr bevorzugt Galactose, und Glucose.

Das Verfahren zur Herstellung des Kohlensäure-Addukts (SKA3) umfasst den Schritt h), Einfrieren der nach Abschluss des Schrittes g3) erhaltenen Lösung.

Der Gehalt an CO₂ in der Lösung, die dem Schritt h) unterzogen wird, mindestens 6 g/l, vorzugsweise mindestens 10 g/l, mehr bevorzugt mindestens 12 g/l, noch mehr bevorzugt mindestens 14g/l und ganz besonders bevorzugt mindestens 15 g/l beträgt. Die Lösung die dem Schritt h) unterzogen wird, wird falls erforderlich mit weiterem CO₂ angereichert bis die vorstehend angegebenen Konzentrationen erreicht werden.

Das Verfahren zur Herstellung des stabilisierten Kohlensäure-Addukts (SKA3) umfasst den Schritt j), Lagerung der in Schritt h) eingefrorenen Lösung bei -100 bis 0 °C für nicht länger als 4 Tage.

Das Verfahren zur Herstellung des stabilisierten Kohlensäure-Addukts (SKA4) umfasst den Schritt k), Trocknung einer mit CO₂ gesättigten Lösung, die das Kohlensäure-Addukt (KA) aus Schritt f) und Polyvinylpyrrolidin umfasst.

Wobei die CO₂ gesättigte Lösung, die in Schritt k) getrocknet wird, eine Konzentration an CO₂ von mindestens 1,2 g/l CO₂, mehr bevorzugt von mindestens 1,5 g/l CO2 beträgt.

In einer Ausführungsform umfasst Schritt k) die Teilschritte
k1) Lösen von Polyvinylpyrrolidon in Wasser unter Erhalt der Lösung (Lk1);
optional k2) Anreicherung der Lösung (Lk1) mit CO₂; Vorzugsweise erfolgt die Anreicherung der Lösung (Lk1) mit CO₂ in Schritt k2) unter Normaldruck.
k3) Lösen des Kohlensäure-Addukts (KA) in der in Schritt k2) oder k1) erhaltenen Lösung (Lk1) unter Erhalt der Lösung (Lk3); vorzugsweise beträgt das Massenverhältnis von Kohlensäure-Addukts (KA) zu Polyvinylpyrrolidon 1:1,5.
k4) Sättigung der Lösung (Lk3) mit CO₂. Wobei die Sättigung vorzugsweise unter Überdruck, bis zu einer Konzentration von mindestens 1,2g/l CO₂.

In einer weiteren Ausführungsform umfasst Schritt k) die Teilschritte
k1) Lösen von Polyvinylpyrrolidon in Wasser unter Erhalt der Lösung (Lk1);
k5) Lösen des Kohlensäure-Addukts (KA) in mit CO₂ gesättigtem Wasser unter Erhalt der Lösung (Lk4);

Die Konzentration des Polyvinylpyrrolidons in Wasser in Schritt k1) in der Lösung (Lk1) beträgt ≤ 850g /l.

Die vorstehend beschriebenen Verfahren zur Herstellung der stabilisierten Kohlensäure-Addukte (SKA1), (SKA2), (SKA3) und (SKA4) sind ein weiterer Aspekt der Erfindung.

### Formulierungen

Die Erfindung umfasst auch pharmazeutische Zubereitungen im Rahmen dieser Erfindung wird grundsätzlich eine Zusammensetzung verstanden, die das stabilisierte Kohlensäure-Addukt (SKA1), (SKA2), (SKA3) oder (SKA4) umfasst und darüber hinaus, weitere Hilfsstoffe oder Zusätze umfassen kann, die für eine pharmazeutisch-medizinische Verwendung geeignet sind.

Außerdem können die erfindungsgemäßen pharmazeutischen Zubereitungen weitere Basen umfassen, die nicht dem Amin (AM) entsprechen und verschieden von der Base (BA) sein können. Der Fachmann kann die Additive grundsätzlich je nach gewünschtem Verwendungszweck auswählen. Er wird dabei die gewünschte Applikationsform berücksichtigen.

Die pharmazeutischen Zubereitungen können grundsätzlich in jeder geeigneten Darreichungsform vorliegen. So können die pharmazeutischen Zubereitungen beispielsweise in Kapselform, als Tablette, als Dragees, als Lyophilisat, als Globuli velati, als Lösung, als Tauchlösung für Wundauflagen, als Salbe, Creme, als Gel, als Paste, Umschlagpaste oder wirkstoffhaltiges Pflaster vorliegen.

Die pharmazeutischen Zubereitungen können grundsätzlich in jeder geeigneten Applikationsform appliziert werden. Der Fachmann wird eine geeignete Darreichungsform entsprechend der beabsichtigten Applikationsform auswählen. Beispielsweise können die pharmazeutischen Zubereitungen parenteral, lingual, sublingual, buccal, oral, inhalativ, per Injektion, als Pflaster, kutan umfassend mindestens die dermale Anwendung, die Anwendung am Auge, die nasale Anwendung, die rektale Anwendung und die vaginale Anwendung; verabreicht werden. Vorzugsweise werden die pharmazeutischen Zubereitungen buccal, inhalativ, parenteral oder oral verabreicht.

Bei der Zubereitung der pharmazeutischen Zubereitungen kann sich der Fachmann grundsätzlich der im Stand der Technik bekannten Methoden bedienen.

Vorzugsweise beträgt die Temperatur der Mischung aus dem Kohlensäure-Addukten (SKA1), (SKA2), (SKA3), oder (SKA3) und den verwendeten Hilfsstoffen und gegebenenfalls weiteren Basen während der Zubereitung der pharmazeutischen Zubereitungen (PZ) weniger als 60 °C, vorzugsweise weniger als 50 °C, mehr bevorzugt 0 bis 50 °C.

Bei der Zubereitung der pharmazeutischen Zubereitungen, optional in Salbenform, kann auch Dispergieren, vorzugsweise mittels eines Salbenbereiters, zum Einsatz kommen. Hierbei wird vorzugsweise eine Drehzahl von < 2000 rpm verwendet.

Die pharmazeutischen Zubereitungen können einen oder mehrere Konservierungsstoffe enthalten. Vorzugsweise ausgewählt aus der Gruppe bestehend aus Methylhydroxybenzoat, Propylhydroxybenzoat, Kaliumsorbat, Natriumsorbat, Sorbinsäure, 4-Hydroxybenzoesäuremethylester, 4-Hydroxybenzoesäurepropylester,

Die stabilisierten Kohlensäure Addukte (SKA1), (SKA3) und (SKA4), vorzugsweise (SKA3) können in einer Ausführungsform als Salbe mit wasserhaltiger Grundlage formuliert wird. Die Salben mit wasserhaltiger Grundlage können grundsätzlich nach allgemein bekannten Grundsätzen hergestellt werden.

Die Salben mit wasserhaltiger Grundlage umfassen vorzugsweise mindestens einen der Hilfsstoffe ausgewählt aus der Gruppe bestehend aus Hydroxyethylcellulosegel, Hydroxyethylcelluloseschleim, Sorbitanmonooleat, Glycerolmonooleat, (C₁₀-C₂₅)alkylOH, Ceteareth-80, Glycerylstearate, Cetearylalkohol und Natriumcetylstearylsulfat.

Das Kohlensäure-Addukt (SKA1), (SKA3), oder (SKA4) kann vor der Verarbeitung zu den nachfolgend beschriebenen oralen Darreichungsformen wie Tabletten oder Kapseln oder halbfesten Darreichungsformen, alleine oder in der Anwesenheit weiterer Hilfsstoffe oder Basen zu Pulver verrieben werden. Der Fachmann kann sich grundsätzlich der für den jeweiligen Zweck geeigneten und bekannten technischen Mittel bedienen. So können für Zerreibungsschritte beispielsweise Mörser, Planetenmühlen oder ähnlich geeignete Geräte eingesetzt werden. Vorzugsweise wird bei den Verreibungsschritten ein technisches Hilfsmittel eingesetzt, dass die mechanische Belastung der stabilisierten Kohlensäure-Addukte möglichst gering hält. Vorzugsweise erfolgt das Zerreiben mit einem Mörser. Vorzugsweise werden die Kohlensäure-Addukte (SKA3) and (SKA4) in einer Planetenmühle zerrieben.

Das so erhaltene Pulver kann dann zum Beispiel zu Tabletten gepresst oder in handelsübliche Kapseln abgefüllt oder mit geeigneten Hilfsstoffen vermischt und zu halbfesten Darreichungsformen verarbeitet werden.

In einer Ausführungsform kann das stabilisierte Kohlensäure Addukt (SKA3), vorzugsweise nach Schritt f) direkt in Tablettenform formuliert bzw. verpresst werden, vorzugsweise ohne das weitere Hilfsstoffe zur Unterstützung der Formulierung der Tablettenform erforderlich sind. Vorzugsweise wird in dieser Ausführungsform in Schritt g3) Polyvinylpyrrolidon (PVP) mit einem durchschnittlichem Molekulargewicht von 24000 bis 33000 g/mol, mehr bevorzugt von 17000 g/mol bis 30000 g/mol verwendet.

Eine Ausführungsform der pharmazeutischen Zubereitung betrifft eine pharmazeutische Zubereitung, die das Kohlensäure-Addukt (SKA1), (SKA3), (SKA4) umfasst, und oral appliziert wird. In dieser Ausführungsform wird die pharmazeutische Zubereitung vorzugsweise in Kapseln, mehr bevorzugt in Hartgelatine oder Cellulose-Kapseln, besonders bevorzugt in Hartgelatine Kapseln verabreicht. Ebenso kann die pharmazeutische Zubereitung in dieser Ausführungsform in Tablettenform appliziert werden.

Vorzugsweise umfasst die pharmazeutische Zubereitung in dieser Ausführungsform mindestens ein Hilfsstoff (H), vorzugsweise ausgewählt aus Stärke, insbesondere Maisstärke und/oder Reisstärke, Dextran, Celluloseester und SiO₂.

Die stabilisierten Kohlensäure-Addukte (SKA1) und (SKA2) wie in Schritt g1) und g2) in Form einer Lösung erhalten, können in einer Ausführungsform direkt als Lösung, vorzugsweise zur oralen Anwendung oder als Spray verwendet werden. In dieser stabilisierten Form als (SKA1) und als (SKA2) können die Kohlensäure-Addukt (KA) aus dem entsprechenden Vorratsgefäss wiederholt entnommen werden, so dass ein Mehrentnahmesystem realisiert werden kann.

Das stabilisierte Kohlensäure-Addukte (SKA2) wie in Schritt g2) in Form einer Lösung erhalten, kann in einer Ausführungsform direkt als Lösung, vorzugsweise zur inhalativen Applikation,als Trinklösung, oder als Parenteralia verwendet werden.

Die vorstehend beschriebenen Verfahren zur Herstellung der stabilisierten Kohlensäure-Addukte (SKA1), (SKA2), (SKA3) und (SKA4) sind ein weiterer Aspekt der Erfindung.

Weiterhin umfasst die Erfindung ein Kit .

Das Kit umfasst:
a) Kohlensäure-Addukt (KA) hergestellt wie für (SKA1) definiert oder in WO2019048590 (A1) offenbart.
b) Polyvinylpyrrolidon;
optional c): eine isotonisch Lösung oder nicht isotonische Lösung, vorzugsweise eine isotonische Lösung ausgewählt aus 0,9 % (w/w) NaCl Lösung, oder einer Vollelektrolytlösung vorzugsweise ausgewählt aus Stereofundin, Stereofundin ISO, Ringerlösung, Ringer-Acetat, Ringer-Lactat, Deltajonin, Jonosteril, und Jonosteril-Malat,
wobei das Kohlensäure-Addukt (KA) und das Polyvinylpyrrolidon optional als Feststoffgemisch vorliegt.

Die Mengenverhältnisse in dem Feststoffgemisch sind wie für (SKA1).

Vorzugsweise ist eine Vollelektrolytlösung eine isotonische Lösung, die in ihrer Zusammensetzung dem Elektrolytprofil des menschlichen Blutplasmas ähnlich ist, und die gleiche Osmolarität wie das Blutplasma besitzt. Eine Vollelektrolytlösung umfasst vorzugsweise mindestens ein Kation und ein Anion, mehr bevorzugt alle der folgenden Ionen: Na⁺, K⁺, Cl⁻, Ca²⁺, Mg²⁺, Acetat, Malat, Laktat. Die Konzentration der Ionen in der Vollelektrolytlösung ist so eingestellt, dass die Vollelektrolytlösung, wie oben aufgeführt, isoton ist.

### Medizinische Anwendungen

Die stabilisierten Kohlensäure-Addukte (SKA1), (SKA2), (SKA3) und (SKA4) bzw. oben genannte Formulierungen sind zur Verwendung in der Medizin anwendbar.

Weiterhin sind die stabilisierte Kohlensäure Addukt (SKA1), (SKA2), (SKA3) und (SKA4) zur Verwendung in einem Verfahren zur Anästhesie, zur Analgesie, zur begleitenden Behandlung von Krebs, zur Behandlung von Amyotropher Lateralsklerose (ALS), zur Entzündungshemmung, zur Unterstützung der Wundheilung, insbesondere bei Verbrennungen, offenen Wunden und Narben, zur Behandlung von neurogenen Entzündungen wie Multiple Sklerose, MMN (Multifokale motorische Neuropathie), zur Behandlung von Sinusitis, zur Behandlung von Asthma, zur Behandlung von Rheumatischer Arthritis, zur Behandlung von Alzheimer, zur Behandlung von Demenz, zur Unterstützung der Rekonvaleszenz und zur Unterstützung des Anti-aging, zur Behandlung des Burn-out-Syndroms, zur Behandlung von Arthrose, zur Behandlung von Polyarthritis, zur Behandlung von Schmerzsyndrom und allgemeinen Schmerzen, zur prä- und postoperativen Behandlung (auch bei Knochenbrüchen), zur Präventiv- und Rehabilitationsmedizin, zur Behandlung von Zosterneuralgie, zur begleitenden Behandlung von Zosterneuralgie, zur Behandlung von Erkrankungen der Bauchorgane, wie Leber, Galle, Bauchspeicheldrüse, Darm, zur Behandlung von Magen-Darm-Erkrankungen (Collitis Ulcerosa, Morbus Krohn), zur Behandlung von Morbus Bechterew, zur Behandlung von chron. Schmerzen des Bewegungsapparats, zur Behandlung von Diabetes (Verbesserung der Blutzuckerwerte), zur Reduktion von Ödemen, Fatique, Long-COVID, zur Verbesserung der Mikrozirkulation und zur Comedikation zu Opioden anwendbar.

### BEISPIELE

### 1. Ausführungsbeispiel 1, Beispiel für die Herstellung einer Ausführungsform des stabilisierten Kohlensäure-Adduktes (SKA3)

### 1.1 Materialien:

- Amin (AM) 64,99 g Procainhydrochlorid (z.B. reinst, zur Verwendung als pharmazeutischer Wirkstoff; Ph. Eur. oder in einer Qualität, die für diesen Zweck geeignet ist)
- Base (BA): 20,01 g Natriumhydrogencarbonat (z.B. reinst oder in einer Qualität, die für diesen Zweck geeignet ist),
- PVP: 127,5 g PVP (PVP= Polyvinylpyrrolidon, auch Polyvidon oder Povidon genannt)
- Lösungsmittel: 530 bis 850 ml Wasser (Aqua ad iniectiabilia)
- CO₂: mind. 7,5 g/l Kohlendioxid aus Druckgasstahlflaschen (CO₂ in geeigneter Qualität)
- Trockeneis zur Bereitung von Kältemischungen und zur Kühlung
- Methanol, techn. zur Bereitung von Kältemischungen

### 1.2 Schritt a)

In eine gereinigte Kunststoffdruckflasche wird Wasser (z.B. Aqua ad iniectiabilia) bis zur Markierung eingefüllt (ca. 800 bis 900 ml) und für mindestens 1h im Kühlschrank (3 bis 8 °C) oder mittels externer Kühlung auf 5 °C vorgekühlt.

Es wird eine mit Kohlendioxid gesättigte Kohlensäurelösung bereitet. Dafür wird CO₂ intervallweise unter Druck (1,6 bis 8 bar) in das vorgekühlte Wasser eingebracht. Das Zischen (entweichendes Gas über das Überdruckventil) zeigt Sättigung der Lösung mit CO₂ an. Die Sättigung wird über das Gewicht kontrolliert, bis 4,0 bis 6,0 g CO₂ (entsprechend 4,5 bis 7,5 g/l) gelöst sind. Die gesättigte Lösung weist einen pH-Wert von ≤ 4,3 bis 4,8 auf. Dieses Kohlensäure-haltige Wasser wird unmittelbar verschlossen sowie mindestens für 1h im Kühlschrank aufbewahrt.

### 1.3 Schritt b)

In einer zweiten Kunststoffdruckflasche wird 20,01 g Natriumhydrogencarbonat vorgelegt, mit 265 ml gekühltem CO₂₋haltigen Wasser versetzt und unter Schwenken aufgelöst.

### 1.4 Schritt c)

Zu dieser Lösung wird die äquivalente Menge an festem Procainhydrochlorid bei gleichbleibender Temperatur gegeben, wobei sich eine nahezu neutrale Lösung bildet, die nach Zugabe von weiteren 265 ml kalten kohlensäure-haltigen Wasser eine klare schwach saure Lösung ergibt. Die Lösung wird mit CO₂ angereichert. Die so bereitete Lösung wird für mindestens eine 1h im Kühlschrank gelagert.

### 1.5 Schritt gx)

In einer dritten Kunststoffdruckflasche mit Magnetrührfisch wird die PVP-Lösung bereitet. Dazu werden 150ml Wasser (Aqua ad iniectabilia) vorgelegt und portionsweise 127,5g PVP zugegeben. Die Rührgeschwindigkeit wird den Gegebenheiten angepasst. Die vollständige Auflösung wird kontrolliert. Die Lösung muss klar sein.

### 1.6 Schritt g3)

Für die Bereitung der Endreaktionslösung wird in die dritte Kunststoffdruckflasche, die die PVP-Lösung enthält, die ProcCluster-Lösung hinzugegeben und so beide Lösungen vereinigt.

Anschließend wird die Lösung nochmals mit CO₂ konditioniert, bis eine CO₂-Konzentration von mind. 7 g/l in der Lösung erreicht ist. Die Lösung wird dann für mindestens 1 h im Kühlschrank gelagert, maximal bis zu 5 Tage.

### 1.5 Schritt d)

Rundkolben werden vorgekühlt. Zum Einfrieren wird die Reaktionslösung in einem vorgekühlten Messzylinder abgemessen, portionsweise in Rundkolben überführt und durch Eintauchen in eine Trockeneis/Methanol-Kältemischung (<-60°C) nach dem Shell-freezing-Verfahren innerhalb von 1,5-3,5 min pro Kolben eingefroren (-200 rpm). Der Eintauchwinkel des Kolbens am Rotationsverdampfer wird auf ca. 40° eingestellt.

### 1.6 Schritt e)

Die Kolben mit dem so eingefrorenen Gut werden mit einem Schliffstopfen verschlossen und einem Tiefkühlschrank bei -15 bis -20 °C 2 bis 4 Tage zwischengelagert.

### 1.7 Schritt f)

Die so temperierten Kolben werden mit Styroporbehältern ummantelt, die vorgekühlt sind und unverzüglich einzeln an eine evakuierte (0,060 ± 0,01 mbar, ca. -46 °C, Dichtheitsprobe) Gefriertrocknungsanlage, über einen flexiblen Gummikegel angeschlossen. Die Ventilhähne werden vorsichtig geöffnet und die einzelnen Kolben unter Vakuum gesetzt. Abschließend müssen alle Kolben evakuiert sein.

Zur Überwachung des Prozesses werden unten im Styropormantel Temperaturfühler platziert, die während der gesamten Trocknung den gesamten Temperaturverlauf aufzeichnen. Vor dem Start der Lyophilisation zeigen die Temperaturfühler Temperaturen von < -5 °C an.

Während der Lyophilisation beträgt der Druck 0,07 ± 0,02 mbar. Dieser Sublimationsdruck wird innerhalb von 4 h erreicht und während der gesamten Lyophilisationszeit beibehalten. Die Kühlkammer wird während der gesamten Trocknung auf 9 bis 15 °C temperiert. Der Endpunkt der Lyophilisation wird graphisch aus den Temperaturverlaufsaufzeichnungen ermittelt. Die Gesamttrocknungszeit betrug maximal 52 h.

Nachdem die Verdampferkolben sich der Raumtemperatur angepasst haben, wird die Substanz in der Pulverisette für 2-mal je 10sec bei 2000rpm homogenisiert.

Das trockene Lyophilisat wird in ein Braunglasgefäß mit Twist-off-Deckel überführt, und im Kühlschrank bei 0 bis 15 °C gelagert.

### 2. Beispiele für Pharmazeutische Zubereitungen (PZ)

### 2.1 Kapseln und Tabletten

Nachfolgend wird beispielhaft die Zusammensetzung der erfindungsgemäßen pharmazeutischen Zubereitung (PZ) in der Ausführungsform als Kapsel oder Tablette für Procain als Amin (AM) beschrieben. Für die Herstellung der Kapseln können handelsübliche Steckkapseln in den handelsüblichen Größen (5 bis 000) verwendet werden, die mit dem stabilisierten Kohlensäure-Addukt (SKA1, SKA3, SKA4) haltigen Pulver, umfassend Procain als Amin (AM) (Verreibung des Wirkstoffs stabilisiertes Kohlensäure-Addukt (SKA), umfassend Procain als Amin (AM) ggf. mit Zusätzen, Füllstoffen, und Fließregulierungsmittel) befüllt werden.

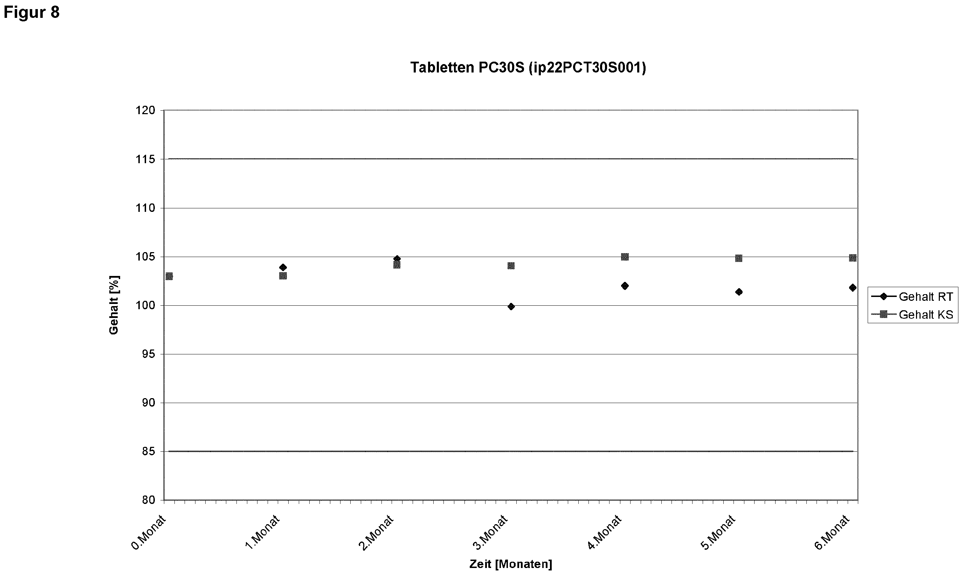

Eine Direkttablettierung wurde ohne Zusätze mit dem homogenisierten Produkt durchgeführt. So wiesen die hergestellten Tabletten beispielhaft mit 190mg Wirkstoff, auch nach 4 Monaten Lagerung im Kühlschrank und bei Raumtemperatur keine Veränderungen auf und sind somit stabil (Figur 8).Die Stabilität wurde mittels IR-Spektroskopie untersucht. So wurde innerhalb des 4 Monatszeitraumes IR-spektroskopisch kein Procain detektiert. Die Gehaltsbestimmung erfolgte bei Raumtemperatur mit UV/VIS-Spektroskopie. Für Rezepturarzneimittel wird gemäß *Pharmacopoea Europaea* Punkt 2.9.6 bezogen auf den Gesamtgehalt inklusive Nebenprodukte ein Toleranzbereich von ±15 % vorgeschrieben.

### 2.2 Salben

Nachfolgend wird die Zusammensetzung der erfindungsgemäßen pharmazeutischen Zubereitung (PZ) in der Ausführungsform als Salbe beispielhaft für Procain als Amin (AM) im stabilisierten Kohlensäure-Addukt (SKA1, SKA3, SKA4) erläutert. Bei der Bereitung der Salbe werden die geltenden und allgemein üblichen pharmazeutischen Regeln zur Herstellung von (Rezeptur)-Arzneimitteln angewendet (zum Beispiel Pharmacopoea Europaea, Deutscher Azneimittel-Codex). Bei der Herstellung der Salbe werden größere Scherkräfte vermieden. Außerdem wird die Temperatur bei der Zubereitung auch lokal unter 60 °C gehalten. So wird das gemörserte stabilisierte Kohlensäure-Addukt (SKA1) bzw. stabilisierte Kohlensäure-Addukt (SKA3, SKA4) umfassend Procain als Amin (AM) in einem Mörser oder einer Fantaschale, die mittels eines auf 40 bis 45 °C temperierten Wasserbades, temperiert werden, in die Salbengrundlage, beispielsweise Vaseline, eingebracht. Alternativ ist auch die Verwendung von elektrischen Mischsystemen möglich, wie sie im Rahmen des üblichen Apothekenbetriebes zum Einsatz kommen.

Als Salbengrundlage können auch z.B. Basiscreme, Wollwachs oder Hydrogele verwendet werden. Eine Temperierung bei der Bereitung ist nicht erforderlich.

Bei der Verwendung von SKA3 oder SKA4 ist ein vorheriges Verreiben nicht notwendig.

### 2.3 buccale, linguale, sublinguale Applikation

Nachfolgend wird die Zusammensetzung der erfindungsgemäßen pharmazeutischen Zubereitung (PZ) in der Ausführungsform als buccale, linguale, sublinguale Applikation beispielhaft für Procain als Amin (AM) im stabilisierten Kohlensäure-Addukt (SKA1, SKA3, SKA4) erläutert. Bei der Bereitung der Applikationen werden die geltenden und allgemein üblichen pharmazeutischen Regeln zur Herstellung von (Rezeptur)-Arzneimitteln angewendet (zum Beispiel Pharmacopoea Europaea, Deutscher Azneimittel-Codex).

### SKA1: Kohlensäureaddukt (KA) + PVP=1:3/1:5 --> mischen --> in Sprayflasche --> direkt vor erster Anwendung lösen

285mg (Kohlensäure-Addukt (KA)) und 852mg PVP25 (Massenverhältnis 1:3) in 5ml CO₂-Wasser (z.B. handelsübliches Mineralwasser, CO₂₋) lösen und mittels Pumpsprayflasche applizieren. PVP= Polyvinylpyrrolidon, auch Polyvidon oder Povidon genannt.

Andere PVP-Produkte sind einsetzbar zum Beispiel PVP30, PVP25 und PVP17 --> andere Verhältnisse (sinnvoll von 1:1 bis 1:10 bezogen auf Menge ProcCluster, gilt für alle verwendeten PVP).

Die Herstellung des Kohlensäure-Addukts (KA) ist in WO2019048590 (A1) offenbart.

### SKA3/SKA4 --> als Spray in Sprayflasche --> direkt vor erster Anwendung lösen

710mg stabilisiertes Kohlensäure-Addukt (SKA3) oder (SKA4) werden in 5ml CO₂-Wasser (z.B. handelsübliches Mineralwasser, CO₂-haltig) lösen und mittels Pumpsprayflasche applizieren. Diese Menge ist entspricht einer Anwendungsdauer von etwa einer Woche. Die Stabilität der Lösung ist für einen Zeitraum von 9 Tagen nach der Herstellung gegeben.

### Gobuli velati

Herstellung entsprechend Pharm. Eur.; (Nicht für homöopathische Anwendung sondern mit Wirkstoffgehalt.)

### Lingual-Tabletten

Siehe Tablettierung oben mit entsprechender geringerer Wirkstoffmenge

### 2.4 inhaltive Applikation

Nachfolgend wird die Zusammensetzung der erfindungsgemäßen pharmazeutischen Zubereitung (PZ) in der Ausführungsform als inhalative Applikation beispielhaft für Procain als Amin (AM) im stabilisierten Kohlensäure-Addukt (SKA1, SKA3, SKA4) erläutert. Bei der Bereitung der Applikationen werden die geltenden und allgemein üblichen pharmazeutischen Regeln zur Herstellung von (Rezeptur)-Arzneimitteln angewendet (zum Beispiel Pharmacopoea Europaea, Deutscher Azneimittel-Codex).

### SKA1: Kohlensäure-Addukt (KA) + PVP=1:3 --> mischen --> in z.B. Bördelrandflasche --> direkt vor erster Anwendung lösen

25mg (Kohlensäure-Addukt (KA)) und 75mg PVP25 (Massenverhältnis 1:3) in 5ml 0,9% Natriumchloridlösung gelöst. PVP= Polyvinylpyrrolidon, auch Polyvidon oder Povidon genannt. Die Lösung wird in einen Vernebler z.B. pariboy gegeben und wie in der Anleitung des Gerätes beschrieben appliziert z.B. über Maske oder Mundstück.

Andere PVP-Produkte sind einsetzbar zum Beispiel PVP30, PVP25 und PVP17 --> andere Verhältnisse (sinnvoll von 1:1 bis 1:10 bezogen auf Menge ProcCluster, gilt für alle verwendeten PVP)

### SKA3/SKA4 --> in Bördelrandflasche --> direkt vor Anwendung

37,5mg stabilisiertes Kohlensäure-Addukt in 5ml Wasser lösen und lösen in einem Vernebler z.B. pariboy gegeben und wie in der Anleitung des Gerätes beschrieben appliziert z.B. über Maske oder Mundstück.

Die Stabilität der Lösung ist für einen Zeitraum von 24h nach der Herstellung gegeben.

### 2.5 parenterale Applikation

Nachfolgend wird die Zusammensetzung der erfindungsgemäßen pharmazeutischen Zubereitung (PZ) in der Ausführungsform als parenterale Applikation beispielhaft für Procain als Amin (AM) im stabilisierten Kohlensäure-Addukt (SKA1, SKA2, SKA3, SKA4) erläutert. Bei der Bereitung der Applikationen werden die geltenden und allgemein üblichen pharmazeutischen Regeln zur Herstellung von (Rezeptur)-Arzneimitteln angewendet (zum Beispiel Pharmacopoea Europaea, Deutscher Azneimittel-Codex).

### SKA1: PC + PVP=1:3 --> mischen --> in z.B. Bördelrandflasche --> direkt vor Anwendung lösen

100mg ProcCluster (Kohlensäure-Addukt) und 300mg PVP25 (Massenverhältnis 1:3) in 5ml Wasser (Aqua ad iniectabilia) oder 0,9% Natriumchloridlösung oder Sterofundin ISO-Lösung gelöst. PVP= Polyvinylpyrrolidon, auch Polyvidon oder Povidon genannt. Diese Lösung wird mittels Sterilfilter (z.B. PES) sterilfiltriert, in 100ml Wasser (Aqua ad iniectabilia) oder 0,9% Natriumchloridlösung oder Sterofundin ISO-Lösung gegeben und direkt als Infusionslöung appliziert.

Die Stabilität der Feststoffmischung ist für einen Zeitraum von 6 Monaten bei Lagerung im Kühlschrank und bei Raumtemperatur gegeben. Die Stabilität der Lösung ist für einen Zeitraum von 24h nach der Herstellung gegeben.

### SKA1: Kohlensäure-Addukt (KA) + PVP=1:3 als Lösung

2g Kohlensäure-Addukt (KA) und 6g PVP25 (Massenverhältnis 1:3) in 200ml CO₂-gesättigten Wasser Wasser (Aqua ad iniectabilia) gelöst und nochmals mit CO₂ gesättigt. PVP= Polyvinylpyrrolidon, auch Polyvidon oder Povidon genannt.

Diese Lösung wird sterilfiltriert und in Bördelrandflaschen abgefüllt. Die Stabilität der Lösung ist für einen Zeitraum von 12 Monaten nach der Herstellung gegeben.

Die Herstellung des Kohlensäure-Addukts (KA) ist in WO2019048590 (A1) offenbart.

### SKA2: (Kohlensäure-Addukt (KA) +NaHCO₃) + PVP = 1:5

### 1.1 Materialien:

- Amin (AM) 48,93 g Procainhydrochlorid (z.B. reinst, zur Verwendung als pharmazeutischer Wirkstoff; Ph. Eur. oder in einer Qualität, die für diesen Zweck geeignet ist)
- Base (BA): 15,07 g Natriumhydrogencarbonat (z.B. reinst oder in einer Qualität, die für diesen Zweck geeignet ist),
- 22,5g PVP 25
- Lösungsmittel: 450 ml Wasser (Aqua ad iniectiabilia)
- CO₂: mind. 7,5 g/l Kohlendioxid aus Druckgasstahlflaschen (CO₂ in geeigneter Qualität)
- Trockeneis zur Bereitung von Kältemischungen und zur Kühlung
- Methanol, techn. zur Bereitung von Kältemischungen

### 1.2 Schritt a)

In eine gereinigte Kunststoffdruckflasche wird Wasser (z.B. Aqua ad iniectiabilia) bis zur Markierung eingefüllt (ca. 400 ml) und für mindestens 1h im Kühlschrank (3 bis 8 °C) oder mittels externer Kühlung auf 5 °C vorgekühlt.

Es wird eine mit Kohlendioxid gesättigte Kohlensäurelösung bereitet. Dafür wird CO₂ intervallweise unter Druck (1,6 bis 8 bar) in das vorgekühlte Wasser eingebracht. Das Zischen (entweichendes Gas über das Überdruckventil) zeigt Sättigung der Lösung mit CO₂ an. Die Sättigung wird über das Gewicht kontrolliert, bis 2,0 bis 4,0 g CO₂ (entsprechend 4,5 bis 7,5 g/l) gelöst sind. Die gesättigte Lösung weist einen pH-Wert von ≤ 4,3 bis 4,8 auf. Dieses Kohlensäure-haltige Wasser wird unmittelbar verschlossen sowie mindestens für 1h im Kühlschrank aufbewahrt.

### 1.3 Schritt b)

In einer zweiten Kunststoffdruckflasche wird 15,07 g Natriumhydrogencarbonat vorgelegt, mit 200 ml gekühltem CO₂₋haltigen Wasser versetzt und unter Schwenken aufgelöst.

### 1.4 Schritt c)

Zu dieser Lösung wird die äquivalente Menge an festem Procainhydrochlorid bei gleichbleibender Temperatur gegeben, wobei sich eine nahezu neutrale Lösung bildet, die nach Zugabe von weiteren 200 ml kalten kohlensäure-haltigen Wasser eine klare schwach saure Lösung ergibt. Die Lösung wird mit CO₂ angereichert. Die so bereitete Lösung wird für mindestens eine 1h im Kühlschrank gelagert.

### 1.5 Schritt g2)

In einer dritten Kunststoffdruckflasche mit Magnetrührfisch wird die PVP-Lösung bereitet. Dazu werden 200ml Wasser (Aqua ad iniectabilia) vorgelegt und 22,5g PVP zugegeben. Die Rührgeschwindigkeit wird den Gegebenheiten angepasst. Die vollständige Auflösung wird kontrolliert. Die Lösung muss klar sein. Zu dieser Lösung werden 28,2ml der zuvor bereiteten Lösung aus Schritt c) gegeben und diese Mischung auf 450ml mit Wasser (Aqua ad iniectabilia) aufgefüllt. Anschließend wird die Lösung nochmals mit CO₂ konditioniert.

Diese Lösung wird sterilfiltriert und in Bördelrandflaschen abgefüllt. Die Stabilität der Lösung ist für einen Zeitraum von 12 Monaten nach der Herstellung gegeben.

### SKA3/SKA4: hergestellt aus Lyophilisat das Galaktose umfasst

10g stabilisiertes Kohlensäure-Addukt Lyophilisat (SKA3) oder (SKA4) umfassend Galaktose (Gewichtsverhältnis Kohlensäure-Addukt:PVP17:Galaktose = 1:3:1) werden in 200ml CO₂-gesättigten Wasser Wasser (Aqua ad iniectabilia) gelöst und nochmals mit CO₂ gesättigt. Diese Lösung wird sterilfiltriert und in Bördelrandflaschen abgefüllt. Die Stabilität der Lösung ist für einen Zeitraum von 12 Monaten nach der Herstellung gegeben.

## Patentansprüche

1. Stabilisiertes Kohlensäure-Addukt (SKA1) umfassend Kohlensäure, mindestens ein Amin (AM) und optional mindestens ein Salz (S),
herstellbar nach einem Verfahren umfassend die Schritte
a) Bereitstellung einer Lösung (A), die mindestens ein Lösungsmittel und in dem mindestens einem Lösungsmittel gelöstes CO₂ umfasst,
optional b) Auflösen einer Base (BA), die nicht dem Amin (AM) entspricht, in der Lösung (A) unter Erhalt der Lösung (A1),
c) Lösen des mindestens einen Amins (AM) in der Lösung (A) oder (A1), unter Erhalt der Lösung (B),
d) Einfrieren der nach Abschluss des Schrittes c) erhaltenen Lösung,
e) Lagerung der in Schritt d) eingefrorenen Lösung bei -100 bis 0 °C für nicht länger als 4 Tage,
g1) Zugabe von Polyvinylpyrrolidon zu dem in den vorangehenden Schritten erhaltenen Kohlensäure-Addukt (KA) unter Erhalt des stabilisierten Kohlensäure-Addukts (SKA1),
wobei der Gehalt an CO₂ in der Lösung, die dem Schritt d) unterzogen wird, mindestens 6 g/l, vorzugsweise mindestens 7 g/l, mehr bevorzugt mindestens 8 g/l, besonders bevorzugt 8,1 g/l bis 9,5 beträgt und das Amin (AM) auch in Form eines Salzes eingesetzt werden kann.

2. Stabilisiertes Kohlensäure-Addukt (SKA2) umfassend Kohlensäure, mindestens ein Amin (AM) und optional mindestens ein Salz (S),
herstellbar nach einem Verfahren umfassend die Schritte
a) Bereitstellung einer Lösung (A), die mindestens ein Lösungsmittel und in dem mindestens einem Lösungsmittel gelöstes CO₂ umfasst,
optional b) Auflösen einer Base (BA), die nicht dem Amin (AM) entspricht, in der Lösung (A) unter Erhalt der Lösung (A1),
c) Lösen des mindestens einen Amins (AM) in der Lösung (A) oder (A1), unter Erhalt der Lösung (B),
g2) Zugabe von Polyvinylpyrrolidon zu der in Schritt c) erhaltenen Lösung (B) unter Erhalt des stabilisierten Kohlensäure-Addukts (SKA2),
wobei der Gehalt an CO₂ in der Lösung (B), die dem Schritt c) mindestens 10 g/l, vorzugsweise mindestens 12 g/l, mehr bevorzugt mindestens 14g/l und ganz besonders bevorzugt mindestens 14 to 16 g/l beträgt und das Amin (AM) auch in Form eines Salzes eingesetzt werden kann.

3. Stabilisiertes Kohlensäure-Addukt (SKA3) umfassend Kohlensäure, mindestens ein Amin (AM) und optional mindestens ein Salz (S),
herstellbar nach einem Verfahren umfassend die Schritte
a) Bereitstellung einer Lösung (A), die mindestens ein Lösungsmittel und in dem mindestens einem Lösungsmittel gelöstes CO₂ umfasst,
optional b) Auflösen einer Base (BA), die nicht dem Amin (AM) entspricht, in der Lösung (A) unter Erhalt der Lösung (A1),
c) Lösen des mindestens einen Amins (AM) in der Lösung (A) oder (A1), unter Erhalt der Lösung (B),
g3) Zugabe von Polyvinylpyrrolidon zu der in Schritt c) erhaltenen Lösung (B) unter Erhalt der Lösung (C),
h) Einfrieren der nach Abschluss des Schrittes g3) erhaltenen Lösung,
j) Lagerung der in Schritt h) eingefrorenen Lösung bei -100 bis 0 °C für nicht länger als 4 Tage,
wobei der Gehalt an CO₂ in der Lösung, die dem Schritt h) unterzogen wird, mindestens 7 g/l, vorzugsweise mindestens 7,5 g/l, beträgt und das Amin (AM) auch in Form eines Salzes eingesetzt werden kann,
wobei die Schritte in der oben aufgeführten Reihenfolge a), optional b), c), g3), h) und j) durchgeführt werden.

4. Stabilisiertes Kohlensäure-Addukt (SKA4) umfassend Kohlensäure, mindestens ein Amin (AM) und optional mindestens ein Salz (S),
herstellbar nach einem Verfahren umfassend die Schritte
a) Bereitstellung einer Lösung (A), die mindestens ein Lösungsmittel und in dem mindestens einem Lösungsmittel gelöstes CO₂ umfasst,
optional b) Auflösen einer Base (BA), die nicht dem Amin (AM) entspricht, in der Lösung (A) unter Erhalt der Lösung (A1),
c) Lösen des mindestens einen Amins (AM) in der Lösung (A) oder (A1), unter Erhalt der Lösung (B),
d) Einfrieren der nach Abschluss des Schrittes c) erhaltenen Lösung,
e) Lagerung der in Schritt d) eingefrorenen Lösung bei -100 bis 0 °C für nicht länger als 4 Tage,
f) Trocknung der in Schritt e) gelagerten Lösung unter Erhalt von Kohlensäure-Addukt (KA),
k) Trocknung einer mit CO₂ gesättigten Lösung, die das Kohlensäure-Addukt (KA) aus Schritt f) und Polyvinylpyrrolidin umfasst,
wobei der Gehalt an CO₂ in der Lösung, die dem Schritt d) unterzogen wird, mindestens 6 g/l, vorzugsweise mindestens 7 g/l, mehr bevorzugt mindestens 8 g/l, besonders bevorzugt 8,1 g/l bis 9,5 g/l beträgt und das Amin (AM) auch in Form eines Salzes eingesetzt werden kann.

5. Ein Kit umfassend
a) Kohlensäure-Addukt (KA) hergestellt wie definiert in Anspruch 1;
b) Polyvinylpyrrolidon;
optional c): eine isotonisch Lösung oder nichtisotonische Lösung, vorzugsweise eine isotonische Lösung vorzugsweise ausgewählt aus 0,9 % (w/w) NaCl Lösung, oder einer Vollelektrolylösung vorzugsweise ausgewählt aus Stereofundin, Stereofundin ISO, Ringerlösung, Ringer-Acetat, Ringer-Lactat, Deltajonin, Jonosteril, und Jonosteril-Malat,
wobei das Kohlensäure-Addukt (KA) und das Polyvinylpyrrolidon optional als Feststoffgemisch vorliegt.

6. Das stabilisierte Kohlensäure-Addukt (SKA1), (SKA2), (SKA3), oder (SKA4) gemäß einem der Ansprüche 1 bis 4, und das Kohlensäure-Addukt (KA) in Anspruch 5 gemäß Anspruch 1, wobei Schritt a) mindestens einen der folgenden Teilschritte umfasst:
a1) Kühlen des Lösungsmittels, vorzugsweise Wasser, auf 3 bis 8 °C, vorzugsweise 5 °C und/oder
a2) Einbringen von CO₂ in das Lösungsmittel, vorzugsweise bis zu einer Sättigungskonzentration von 3 bis 10 g/l, mehr bevorzugt bis zu einer Sättigungskonzentration 4,5 bis 7,5 g/l, vorzugsweise beträgt der pH-Wert der Lösung nach der Sättigung mit CO₂ ≤ 3,0 bis 6,0, noch mehr bevorzugt ≤ 4,3 bis 4,8 und/oder
a3) Lagerung der Lösung (A) bei 1 bis 10°C, vorzugsweise für mindestens 30 min, mehr bevorzugt für mindestens 50 min, noch mehr bevorzugt für mindestens 60 min; bis höchstens 5d (120h); vorzugsweise erfolgt die Lagerung bei 3 bis 8 °C vorzugsweise für mindestens 30 min, mehr bevorzugt für mindestens 50 min, noch mehr bevorzugt für mindestens 60 min; bis höchstens 5d (120h);
vorzugsweise umfasst Schritt a) alle Teilschritte a1), a2) und a3),
vorzugsweise werden die Teilschritte a1), a2) und a3) in der Reihenfolge a2) folgt auf a1) und a3) folgt auf a2) durchgeführt.

7. Das stabilisierte Kohlensäure-Addukt (SKA1), (SKA2), (SKA3) oder (SKA4) gemäß einem der Ansprüche 1 bis 4, 6 und das Kohlensäure-Addukt (KA) in Anspruch 5 gemäß Anspruch 1 wobei die Base (BA) in Schritt b) ein Hydrogencarbonat oder ein Carbonat, mehr bevorzugt ein Hydrogencarbonat, noch mehr bevorzugt Natriumhydrogencarbonat ist.

8. Das stabilisierte Kohlensäure-Addukt (SKA1), (SKA2), (SKA3) oder (SKA4) gemäß einem der Ansprüche 1 bis 4, 6, 7 und das Kohlensäure-Addukt (KA) in Anspruch 5 gemäß Anspruch 1, wobei Schritt c) mindestens einen der folgenden Teilschritte umfasst:
c1) Auflösen des mindestens einen Amins (AM) in Lösung (A) oder (A1) unter Erhalt der Lösung (B) und/oder
c2) Zugabe von Lösung (A) zur Lösung (B) unter Erhalt der Lösung (B1) und/oder c3) Anreicherung der Lösung (B) oder (B1) mit CO₂ und/oder
c4) Lagerung der Lösung (B) oder (B1) bei 1 bis 10°C, vorzugsweise 3 bis 8 °C, für mindestens 1 h vorzugsweise 24 h bis 120 h, noch mehr bevorzugt 24 bis 72 h, und/oder
c5) Anreicherung der Lösung (B) oder (B1) mit CO₂ auf eine Konzentration von mindestens 6 g/l, vorzugsweise mindestens 10 g/l, mehr bevorzugt mindestens 12 g/l, noch mehr bevorzugt mindestens 14 g/l und ganz besonders bevorzugt mindestens 15 g/l;
wobei optional
i) die Konzentration des Amins (AM) in Lösung (B) oder bei Ausführung von Teilschritt c2) in Lösung (B1) 0,01 bis 0,25 g/ml, vorzugsweise 0,03 bis 0,20 g/ml, mehr bevorzugt 0,08 bis 0,15 g/ml beträgt und/oder
ii) der pH-Wert der Lösung (B) oder (B1) nach der Durchführung von Schritt c5) ≤ 7,0 beträgt und/oder
iii) das Verhältnis des Amins (AM) zur Base (BA), bei Durchführung des Schrittes b), in Lösung (B) 2:1 bis 5:1, mehr bevorzugt 3:1 bis 4:1, noch mehr bevorzugt 3,23:1 bis 3,26:1 [g/g] beträgt und/oder
iv) in Schritt c1) das mindestens eine Amin (AM), das mindestens eine Amin (AM) als Säureadditionssalz, vorzugsweise als Hydrohalogenid, Hydrogensulfat, Hydrogensulfit, Hydrogenphosphat, Hydromesylat, Hydrotosylat, Hydroacetat, Hydroformiat, Hydropropanoat, Hydromalonat, Hydrosuccinat, Hydrofumarat, Hydroxalat, Hydrotartrat, Hydrocitrat, Hydromaleat, mehr bevorzugt als Hydrochlorid oder Hydrobromid umfasst.
vorzugsweise umfasst Schritt c) alle Teilschritte c1), c2), c3), c4)und c5);
vorzugsweise werden die Teilschritte c1), c2), c3), c4) und c5) in der Reihenfolge c2) folgt auf c1), c3) folgt auf c2), c4) folgt auf c3), c5) folgt auf c4) durchgeführt.

9. Das stabilisierte Kohlensäure-Addukt (SKA1) oder (SKA4) gemäß einem der Ansprüche 1 bis 4, 6, bis 8 und das Kohlensäure-Addukt (KA) in Anspruch 5 gemäß Anspruch 1 wobei in Schritt d):
i) die Lösung (B) oder (B1) bei -100 °C bis -20 °C, vorzugsweise bei -90 °C bis -30 °C, noch mehr bevorzugt bei -80 bis -40 °C und ganz besonders bevorzugt bei -70 bis -50 °C eingefroren wird und/oder
ii) die Lösung (B) oder (B1) innerhalb von 0,3 bis 60 Minuten, vorzugsweise innerhalb von 1 bis 30 Minuten, mehr bevorzugt innerhalb von 1,1 bis 10 Minuten, noch mehr bevorzugt innerhalb von 1,5 bis 5 Minuten eingefroren wird und/oder
iii) das Gefäß in dem sich die Lösung (B) oder (B1) während des Einfriervorgangs befindet, vorzugsweise im Kühlmedium, mit 10 bis 1000 rpm, vorzugsweise mit 50 bis 600 rpm, mehr bevorzugt mit 100 bis 400 rpm und noch mehr bevorzugt mit 200 bis 300 rpm rotiert wird und/oder
iv) die Lösung (B) oder (B1) mit einer Kühlrate von 10 bis 100 K/min, vorzugsweise mit 20 bis 80 K/min, mehr bevorzugt mit 30 bis 70 K/min und besonders bevorzugt mit 40 bis 60 K/min eingefroren wird.

10. Das stabilisierte Kohlensäure-Addukt (SKA1) oder (SKA4) gemäß einem der Ansprüche 1 bis 4, 6 bis 9 und das Kohlensäure-Addukt (KA) in Anspruch 5 gemäß Anspruch 1 wobei in Schritt e):
i) die eingefrorene Lösung (B) oder (B1) für 1,5 bis 4 Tage, vorzugsweise für 2,5 bis 4 Tage gelagert wird und/oder
ii) die eingefrorene Lösung (B) oder (B1) vorzugsweise bei -50 bis 0 °C, mehr bevorzugt bei -30 bis -5 °C, noch mehr bevorzugt bei -25 bis -10 °C, besonders bevorzugt bei -20 bis -15 °C gelagert wird.

11. Das Kohlensäure-Addukt (SKA1), (SKA3), oder (SKA4) gemäß einem der Ansprüche 1 bis 4, 6 bis 10 und das Kohlensäure-Addukt (KA) in Anspruch 5 gemäß Anspruch 1, wobei das Verfahren einen weiteren Schritt f) umfasst, der nach Schritt e) oder j) durchgeführt wird,
f) Trocknung der in Schritt e) oder j) gelagerten Lösung unter Erhalt von getrocknetem Kohlensäure-Addukt (SKA1), oder (SKA3); oder (KA)
wobei in Schritt f) optional
i) das Wasser aus der Lösung (B) oder (B1) bis zu einem Restgehalt von < 0,8 Gew.-%, vorzugsweise < 0,1 Gew.-% bezogen auf das Gesamtgewicht des Trocknungsproduktes (C) entfernt wird und/oder
ii) nicht im Kohlensäure-Addukt (KA) gebundenes CO₂ aus der Lösung (B) oder (B1) bis zu einem Restgehalt von < 0,8 Gew.-%, vorzugsweise < 0,1 Gew.-% bezogen auf das Gesamtgewicht des Trocknungsproduktes (C) entfernt wird und/oder
iii) die Trocknung mittels Lyophilisation durchgeführt wird und/oder
iv) während des Trocknens der Druck 0,01 bis 30 mbar, vorzugsweise 0,02 bis 20 mbar, mehr bevorzugt 0,03 bis 10 mbar, noch mehr bevorzugt 0,03 bis 0,5 mbar und ganz besonders bevorzugt 0,05 bis 0,1 mbar beträgt und vorzugsweise während des gesamten Trocknungsvorganges beibehalten wird und/oder
v) der Druck während des Trocknens gemäß iv) innerhalb von 10 h, vorzugsweise innerhalb von 7 h, mehr bevorzugt innerhalb von 5 h und besonders bevorzugt innerhalb von 4 h ab Evakuierungsbeginn erreicht wird und/oder
vi) die Temperatur während der gesamten Trocknung in Schritt f) 0 bis 20 °C, vorzugsweise 4 bis 18 °C, mehr bevorzugt 8 bis 16 °C beträgt und/oder
vii) die Gesamttrocknungszeit 10 bis 60 h, vorzugsweise 30 bis 55 h, mehr bevorzugt 41 bis 52 h beträgt.

12. Das stabilisierte Kohlensäure Addukt (SKA1), (SKA2), (SKA3) und (SKA4) gemäß einem der Ansprüche 1 bis 4, 6 bis 11 und das Kohlensäure-Addukt (KA) in Anspruch 5 gemäß Anspruch 1, wobei
A) mindestens ein Amin (AM) gemäß einer der nachstehenden Formeln (I) oder (II) eingesetzt wird, 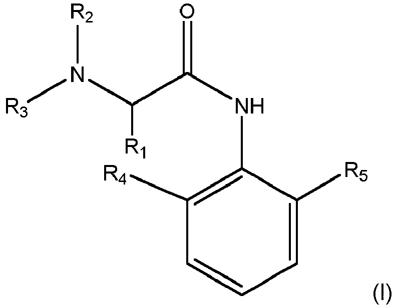 wobei in Formel (I)
R₁ ist H, C₁₋₁₀ Alkyl, C₂₋₁₀ Alkenyl, Aryl oder Heteroaryl, vorzugsweise H oder C₁₋₁₀ Alkyl, mehr bevorzugt H oder C₁₋₄ Alkyl;
R₂ ist H, C₁₋₁₀ Alkyl, C₂₋₁₀ Alkenyl, Aryl oder Heteroaryl, vorzugsweise H oder C₁₋₁₀ Alkyl, mehr bevorzugt H oder C₁₋₄ Alkyl;
R₃ ist H, C₁₋₁₀ Alkyl, C₂₋₁₀ Alkenyl, Aryl oder Heteroaryl, vorzugsweise H oder C₁₋₁₀ Alkyl, mehr bevorzugt H oder C₁₋₄ Alkyl;
wobei optional R₁ und R₃ miteinander verbunden sein können und zusammen mit dem Stickstoffatom an das R₃ gebunden ist und dem Kohlenstoffatom an das R₁ gebunden ist, einen gesättigten oder ungesättigten, vorzugsweise einen gesättigten Ring bilden können, vorzugsweise ist der Ring 4, 5 oder 6 gliedrig, mehr bevorzugt 5 oder 6 gliedrig,
R₄ ist H, Halogen, C₁₋₁₀ Alkyl, C₂₋₁₀ Alkenyl, Aryl oder Heteroaryl, vorzugsweise H oder Methyl;
R₅ ist H, Halogen, C₁₋₁₀ Alkyl, C₂₋₁₀ Alkenyl, Aryl oder Heteroaryl, vorzugsweise H, Methyl oder Halogen; 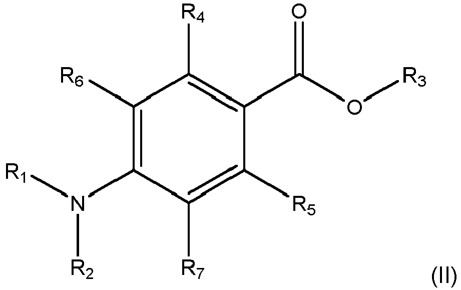 wobei in Formel (II)
R₁ ist H, C₁₋₁₀ Alkyl, C₂₋₁₀ Alkenyl, Aryl oder Heteroaryl, vorzugsweise H oder C₁₋₁₀ Alkyl, mehr bevorzugt H;
R₂ ist H, C₁₋₁₀ Alkyl, C₂₋₁₀ Alkenyl, Aryl oder Heteroaryl, vorzugsweise H oder C₁₋₁₀ Alkyl, mehr bevorzugt H:
R₃ ist H, C₁₋₁₀ Alkyl, C₂₋₁₀ Alkenyl, Aryl, Heteroaryl oder -(CH₂)ₙNR₈R₉, vorzugsweise - (CH₂)ₙNR₈R₉,
n ist 1 bis 5, vorzugsweise 1 bis 3, mehr bevorzugt 1 bis 2,
R₈ ist C₁₋₁₀ Alkyl, vorzugsweise C₁₋₂ Alkyl,
R₉ ist C₁₋₁₀ Alkyl, vorzugsweise C₁₋₂ Alkyl;
R₄ ist H, Halogen, C₁₋₁₀ Alkyl, C₂₋₁₀ Alkenyl, Aryl, Heteroaryl oder -O-C₁₋₁₀ Alkyl, vorzugsweise H, Halogen, C₁₋₁₀Alkyl oder -O-C₁₋₁₀ Alkyl, mehr bevorzugt H oder Halogen;
R₅ ist H, Halogen, C₁₋₁₀ Alkyl, C₂₋₁₀ Alkenyl, Aryl, Heteroaryl oder -O-C₁₋₁₀ Alkyl, vorzugsweise H, Halogen, C₁₋₁₀Alkyl oder -O-C₁₋₁₀ Alkyl, mehr bevorzugt H oder Halogen;
R₆ ist H, Halogen, C₁₋₁₀ Alkyl, C₂₋₁₀ Alkenyl, Aryl, Heteroaryl oder -O-C₁₋₁₀ Alkyl, vorzugsweise H, Halogen, C₁₋₁₀Alkyl oder -O-C₁₋₁₀ Alkyl, mehr bevorzugt H oder -O-C₁₋₁₀ Alkyl;
R₇ ist H, Halogen, C₁₋₁₀ Alkyl, C₂₋₁₀ Alkenyl, Aryl, Heteroaryl oder -O-C₁₋₁₀ Alkyl, vorzugsweise H, Halogen, C₁₋₁₀Alkyl oder -O-C₁₋₁₀ Alkyl, mehr bevorzugt H oder -O-C₁₋₁₀ Alkyl;
wobei das mindestens eine Amin gemäß Formel (I) und (II) optional auch in Form eines Salzes eingesetzt werden kann oder
B) mindestens ein Amin (AM) eingesetzt wird, ausgewählt aus der Gruppe 4-Aminobenzoesäure-2-(*N*,*N*-diethylamino-)ethylester (Procain), 4-Aminobenzoesäureethylester (Benzocain), 2-(Diethylamino)ethyl-4-amino-2-chlorbenzoat (Chlorprocain), 4-Amino-3-butoxybenzoesäure-2-diethylaminoethylester (Oxybuprocain), (2-(Dimethylamino)ethyl)-4-(butylamino)benzoat (Tetracain), N-[3-(4-Phenoxymethylphenyl)propyl]morpholin (Fomocain), 2-Diethylamino-N-(2,6-dimethylphenyl)acetamid (Lidocain), (RS)-N-(2,6-Dimethylphenyl)-1-methylpiperidin-2-carboxamid (Mepivacain), (RS)-N-(2-Methylphenyl)-2-(propylamino)-propanamid (Prilocain), (RS)-4-Methyl-3-[2-(propylamino)-propanamido]thiophen-2-carbonsäuremethylester (Articain), (±)-1-Butyl-N-(2,6-dimethylphenyl)-2-piperidincarboxamid (Bupivacain), (S)-1-Propyl-2',6'-dimethyl-2-piperidylcarboxyanilid (Ropivacain), 2-(Ethylpropylamino)-2',6'-butyroxylidid (Etidocain), 1-(4-Butoxyphenyl)-3-piperidin-1-ylpropan-1-on (Dyclonin), vorzugsweise 4-Aminobenzoesäure-2-(*N*,*N*-diethylamino-)ethylester (Procain) und 2-Diethylamino-*N*-(2,6-dimethylphenyl)acetamid (Lidocain) (2-(Dimethylamino)ethyl)-4-(butylamino)benzoat (Tetracain) und/oder der Salze dieser Verbindungen.

13. Verfahren zur Herstellung
i) des stabilisierten Kohlensäure-Addukts (SKA1) gemäß einem der Ansprüche 1 und 6 bis 12, umfassend die Schritte gemäß einem der Ansprüche 1 und 6 bis 12, bezogen auf das stabilisierte Kohlensäure-Addukts (SKA1); oder
ii) des stabilisierten Kohlensäure-Addukts (SKA2) gemäß einem der Ansprüche 2 und 6 bis 12, umfassend die Schritte gemäß einem der Ansprüche 2 und 6 bis 12, bezogen auf das stabilisierte Kohlensäure-Addukts (SKA2); oder
iii) des stabilisierten Kohlensäure-Addukts (SKA3) gemäß einem der Ansprüche 3 und 6 bis 12, umfassend die Schritte gemäß einem der Ansprüche 3 und 6 bis 12, bezogen auf das stabilisierte Kohlensäure-Addukts (SKA3) oder
iv) des stabilisierten Kohlensäure-Addukts (SKA4) gemäß einem der Ansprüche 4 und 6 bis 12, umfassend die Schritte gemäß einem der Ansprüche 4 und 6 bis 12, bezogen auf das stabilisierte Kohlensäure-Addukts (SKA4).

14. Das stabilisierte Kohlensäure Addukt (SKA1), (SKA2), (SKA3) und (SKA4) gemäß einem der Ansprüche 1 bis 4 und 6 bis 12 zur Verwendung in einem Verfahren zur Anästhesie, zur Analgesie, zur begleitenden Behandlung von Krebs, zur Behandlung von Amyotropher Lateralsklerose (ALS), zur Entzündungshemmung, zur Unterstützung der Wundheilung, insbesondere bei Verbrennungen, offenen Wunden und Narben, zur Behandlung von neurogenen Entzündungen wie Multiple Sklerose, MMN (Multifokale motorische Neuropathie), zur Behandlung von Sinusitis, zur Behandlung von Asthma, zur Behandlung von Rheumatischer Arthritis, zur Behandlung von Alzheimer, zur Behandlung von Demenz, zur Unterstützung der Rekonvaleszenz und zur Unterstützung des Anti-aging, zur Behandlung des Burn-out-Syndroms, zur Behandlung von Arthrose, zur Behandlung von Polyarthritis, zur Behandlung von Schmerzsyndrom und allgemeinen Schmerzen, zur prä- und postoperativen Behandlung (auch bei Knochenbrüchen), zur Präventiv- und Rehabilitationsmedizin, zur Behandlung von Zosterneuralgie, zur begleitenden Behandlung von Zosterneuralgie, zur Behandlung von Erkrankungen der Bauchorgane, wie Leber, Galle, Bauchspeicheldrüse, Darm, zur Behandlung von Magen-Darm-Erkrankungen (Collitis Ulcerosa, Morbus Krohn), zur Behandlung von Morbus Bechterew, zur Behandlung von chron. Schmerzen des Bewegungsapparats, zur Behandlung von Diabetes (Verbesserung der Blutzuckerwerte), zur Reduktion von Ödemen, zur Comedikation zu Opioden.

15. Das stabilisierte Kohlensäure Addukt (SKA1) gemäß einem der Ansprüche 1, 4 und 6 bis 14 oder das Kit gemäß Anspruch 5 wobei
i) in Schritt g1) oder im Feststoffgemisch das Gewichtsverhältnis Polyvinylpyrrolidon (PVP) zum Kohlensäure-Addukt (KA) 10: 1, mehr bevorzugt 5:1, besonders bevorzugt 3:1 beträgt; und/oder
ii) das Polyvinylpyrrolidon (PVP) ein durchschnittliches Molekulargewicht von 10000 bis 40000 g/mol, vorzugsweise von 15000 bis 35000 g/mol, mehr bevorzugt von 24000 bis 33000 g/mol, besonders bevorzugt von 17000 g/mol bis 32000 g/mol aufweist; und/oder
iii) die Applikation parenteral, buccal, inhalativ, oral, sublingual oder lingual erfolgt.

16. Das stabilisierte Kohlensäure Addukt (SKA2) gemäß einem der Ansprüche 2 und 6 bis 14, wobei,
i) in Schritt g2) das Gewichtsverhältnis Polyvinylpyrrolidon (PVP) zum Kohlensäure-Addukt (KA) 20: 1, mehr bevorzugt 10:1, besonders bevorzugt 5:1 beträgt; und/oder
ii) das Polyvinylpyrrolidon (PVP) ein durchschnittliches Molekulargewicht von von 15000 bis 35000 g/mol, mehr bevorzugt von 24000 bis 33000 g/mol, besonders bevorzugt von 16000 g/mol bis 27000 g/mol aufweist; und/oder
iii) die Applikation buccal, inhalativ, parenteral oral, lingual oder sublingual erfolgt.

17. Das stabilisierte Kohlensäure Addukt (SKA3) gemäß einem der Ansprüche 3 und 6 bis 14, wobei,
i) in Schritt g3) das Gewichtsverhältnis Polyvinylpyrrolidon (PVP) zum Kohlensäure-Addukt (KA) 6:1 bis 1:0,5, mehr bevorzugt 3:1, besonders bevorzugt 1,5:1 beträgt; und/oder
ii) das Polyvinylpyrrolidon (PVP) ein durchschnittliches Molekulargewicht von 10000 bis 95000 g/mol, vorzugsweise von 15000 bis 35000 g/mol, mehr bevorzugt von 24000 bis 33000 g/mol, besonders bevorzugt von 17000 g/mol bis 32000 g/mol aufweist; und/oder
iii) die Applikation parenteral, buccal, inhalativ , oral, lingual oder sublingual erfolgt und/oder
iv) das stabilisierte Kohlensäure Addukt (SKA3) in Tablettenform formuliert wird, vorzugsweise bei Verwendung von Polyvinylpyrrolidon (PVP) in Schritt g3) mit einem durchschnittlichem Molekulargewicht von 24000 bis 33000 g/mol, mehr bevorzugt von 17000 g/mol bis 30000 g/mol und/oder
v) das stabilisierte Kohlensäure Addukt (SKA3) als Salbe mit wasserhaltiger Grundlage formuliert wird; und/oder
vi) das stabilisierte Kohlensäure Addukt (SKA3) als Gel formuliert wird; und/oder
vii) in Schritt g3) ein oder mehrere weitere Additive zugesetzt werden, ausgewählt aus einem Monosaccharid, vorzugsweise Galactose, und Glucose und/oder
viii) die Applikation parenteral, buccal, inhalativ, oral, lingual, oder sublingual erfolgt.

18. Das stabilisierte Kohlensäure-Addukt (SKA4) gemäß einem der Ansprüche 4 und 6 bis 14, wobei,
i) in Schritt k) das Gewichtsverhältnis Polyvinylpyrrolidon (PVP) zum Kohlensäure-Addukt (KA) 6: 1 bis 1: 0,5 , mehr bevorzugt 3:1, besonders bevorzugt 1,5:1; ganz besonders bevorzugt 1: 0,5 beträgt und/oder
ii) das Polyvinylpyrrolidon (PVP) ein durchschnittliches Molekulargewicht von 10000 bis 95000 g/mol, vorzugsweise von 15000 bis 35000 g/mol, mehr bevorzugt von 24000 bis 33000 g/mol, besonders bevorzugt von 17000 g/mol bis 32000 g/mol aufweist; und/oder
iii) die Applikation parenteral, buccal, inhalativ, oral, lingual, oder sublingual erfolgt und/oder
iv) das stabilisierte Kohlensäure Addukt (SKA4) als Gel formuliert wird.
